(19) Europäisches Patentamt
European Patent Office
Office européen des brevets

(11) EP 1 866 331 B1

(12) **EUROPEAN PATENT SPECIFICATION**

(45) Date of publication and mention of the grant of the patent:
**20.06.2012 Bulletin 2012/25**

(21) Application number: **06733052.2**

(22) Date of filing: **07.02.2006**

(51) Int Cl.:
***C07K 14/415*** *(2006.01)*

(86) International application number:
**PCT/NL2006/050021**

(87) International publication number:
**WO 2006/085769 (17.08.2006 Gazette 2006/33)**

(54) **METHODS AND MEANS FOR DETERMINING AND CONFERRING STRESS TOLERANCE IN PLANTS**

VERFAHREN UND MITTEL ZUR MESSUNG UND ERZIELUNG VON STRESS TOLERANZ IN PFLANZEN

MÉTHODES ET MOYENS POUR LA DÉTERMINATION ET L'ACQUISITION DE LA TOLÉRANCE AU STRESS DES PLANTES

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.02.2005 EP 05075317**

(43) Date of publication of application:
**19.12.2007 Bulletin 2007/51**

(73) Proprietor: **Nsure Holding B.V.**
**6708 PD Wageningen (NL)**

(72) Inventors:
- **VAN WORDRAGEN, Monique Francisca**
  **NL-6824 NB Arnhem (NL)**
- **BALK, Peter Albert**
  **NL-6671 BD Zetten (NL)**
- **VAN DER GEEST, Apolonia H.m.**
  **NL-6708 KX Wageningen (NL)**

(74) Representative: **Swinkels, Bart Willem**
**Nederlandsch Octrooibureau**
**P.O. Box 29720**
**2502 LS Den Haag (NL)**

(56) References cited:
- **LABHILILI M ET AL: "CHARACTERIZATION OF CDNAS ENCODING TRITICUM DURUM DEHYDRINS AND THEIR EXPRESSION PATTERNS IN CULTIVARS THAT DIFFER IN DROUGHT TOLERANCE" PLANT SCIENCE, LIMERICK, IE, vol. 112, 1995, pages 219-230, XP001016189 ISSN: 0168-9452**
- **LOPEZ CESAR G ET AL: "Dehydrin expression and drought tolerance in seven wheat cultivars." CROP SCIENCE, vol. 43, no. 2, April 2003 (2003-04), pages 577-582, XP002331968 ISSN: 0011-183X**
- **DATABASE EMBL [Online] 24 October 2002 (2002-10-24), "Pinus sylvestris mRNA for dehydrin (dhn5 gene)" XP002331914 retrieved from EBI accession no. EM_PRO:AJ512364 Database accession no. AJ512364**
- **DATABASE EMBL [Online] 24 October 2002 (2002-10-24), "Pinus sylvestris partial mRNA for dehydrin (dhn2 gene)" XP002331915 retrieved from EBI accession no. EM_PRO:AJ512361 Database accession no. AJ512361**
- **CLOSE TIMOTHY J: "Dehydrins: A commonality in the response of plants to dehydration and low temperature" PHYSIOLOGIA PLANTARUM, vol. 100, no. 2, 1997, pages 291-296, XP002331912 ISSN: 0031-9317**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 866 331 B1

## Description

### Field of the invention

The current invention relates to the field of plant biology, breeding and agriculture. The invention also relates to methods of determining gene expression levels, in particular methods in involving quantitative amplification or hybridization to oligonucleotides on solid carriers. The invention provides methods for diagnosing plants for, and producing plants with, enhanced stress tolerance, in particular cold and/or drought tolerance.

### Background of the invention

In plants, expression of many members of the large group of Late Embryogenesis Abundant (LEA) proteins is correlated with desiccation tolerance, and changes in expression are observed in relation to stresses that have a dehydration component. While originally identified in cotton embryos, LEA proteins appeared to be widespread over the plant kingdom and at least six different groups have been identified (Wise and Tunnacliffe 2004). One of the groups comprises the so-called dehydrins (LEA DII family). Dehydrins typically accumulate in plants during dehydration (Close 1996).

Several classes of dehydrins can be distinguished on the basis of structural features. All dehydrins share a highly conserved Lysine-rich sequence, the K-segment (consensus KIKEKLPG). In many dehydrins K-segments are found in more than one copy and in combination with a Serine stretch, the S-segment (consensus SSSSSSSS, usually between 5 and 10 serine residues, mostly 8). The third feature that can be distinguished in many dehydrins is the Y-segment (consensus DEYGNP). S, K and Y segments occur in many different combinations (Campbell and Close 1997, Close 1997). So far, no clear physiological functions have been assigned to dehydrins, although the number of reports that points to specialised functions for individual dehydrins is increasing. Chaperone-like functions have been suggested (Close 1996, Rinne et al. 1999) as well as cryoprotective properties (Momma et al. 2003). Also ion (calcium) binding properties of ERD14 from *Arabidopsis* (Alsheikh et al. 2003) and a dehydrin like protein from *Apium graveolens* (Heyen et al. 2002) have been found. Differences in spatial distribution points to specialised functions (Nylander et al. 2001) as well as reports on associations of dehydrins with lipid vesicles (Koag et al. 2003) and plasmodesmata (Karlson et al. 2003).

Dehydrins have also been cloned from angiosperms (Close 1997) and in most cases, they are members of a multigene family (Choi et al. 1999, Nylander et al. 2001 and Porat et al. 2002). The number of dehydrin genes cloned from gymnosperms is increasing. Several LEA cDNA clones were isolated from seeds of Douglas fir (Jarvis et al. 1996) and a dehydrin cDNA was cloned from needles of white spruce (Richard et al. 2000). Besides these, there are cDNA-libraries from *Pinus taeda* (Johnson 2001, The MIPS database) and *Pinus pinaster*- (Frigerio 2003, NCBI) that contain several dehydrin coding sequences. From *Pinus sylvestris* two genomic fragments have been isolated that encode dehydrins (Acc.no. AF359133 and AF359134).

A link between dehydrin expression and frost tolerance is suspected because of the dehydration that plays a role at low temperature exposure. Plants growing in the temperate zones have to survive cold periods and show an annual growth rythm, dictated by changes in photoperiod and temperature. Decreased daylength is generally assumed to be the trigger for growth cessation (Bigras 1996). As dormancy is always associated with frost tolerance, dehydrins are reported to be related to both phenomena (Rowland and Arora 1997, Artlip et al. 1997). In transgenic aspen (Welling et al. 2002) as well as in birch (Welling et al. 2004) it was shown that both photoperiod / daylength and temperature regulate dehydrin expression.

Plants generally contain several dehydrin sequences, which always comprise at least one K segment and optionally further K, Y or S segments / domains (Campbell and Close, 1997). Although the use of dehydrins in general for determining and conferring cold or drought tolerance in plants is widely acknowledged, little information is available about functional differences between the various types of dehydrins.

The use of dehydrin gene encoded polypeptides for conferring cold tolerance and for determining cold tolerance for selection and breeding purposes has been disclosed in US Patent 6,501,006. In this disclosure a $Y_2K$ type dehydrin sequence cloned from Cowpea is used to confer cold tolerance. The $Y_2K$ dehydrin does not contain a stretch of serines such as SK type dehydrins. $Y_2K$ type dehydrins are not present in all plants. In fact, Y-segment containing dehydrins are absent in many cases (Close 1997), which severely limits the application of $Y_2K$ type dehydrins for conferring cold tolerance and/or determining cold tolerance in such plants.

### Summary of the invention

The current invention exploits a marked difference in the temperature and light regimen induced transcriptional regulation of SK and K-type dehydrins, in order to determine stress tolerance, in particular the cold and/or drought tolerance in a plant, wherein the plant to be tested is a gymnosperm. Tolerance is not determined in absolute terms of tolerance levels, but rather in terms of relative tolerance levels in comparison to other plants of the same genus or species, for instance in a breeding and selection program for this trait, or for determining the timing of induction of stress tolerance or hardening of the plant, for instance for agricultural purposes. Furthermore, the invention provides methods for increasing stress tolerance, in particular cold tolerance, in a plant, wherein the plant to be tested is a gymnosperm by providing a source of a K-type dehydrin. The functional difference between the two types of dehydrins, the K-type dehydrins and SK-type dehydrins, depends on the presence or absence of a serine stretch or S-segments in a dehydrin polypeptide, which is found to have implications for determining and/or conferring stress tolerance, in particular cold or drought tolerance. Preferably the K type and SK type dehydrins disclosed herein do not contain a Y-segment.

Expression of dehydrins is generally associated with cold tolerance in plants and expression of dehydrins is observed to increase during hardening of plants, induced by cold periods or differences in photo-period and -intensity. For instance, Labhilili et al. (Plant Science 112, 219-30, 1995) discloses the characterization of dehydrin species in Triticum durum. Differences in dehydrin expression were detected in drought-tolerant vs. drought-sensitive cultivars. Lopez et al, (Crop Science 43, 577-82, 2003) shows the comparison of expression patterns of dehydrins in seven wheat cultivars. Higher expression of dehydrins is correlated with higher drought tolerance. The current invention discloses a pattern observed in hardening of plants, in particular in gymnosperms, in which during hardening SK-type dehydrins arc first (slowly) induced, at the transcriptional level, followed by a decrease in transcription activity in the cold / winter period, when dormancy is induced. Simultaneously with the decrease of SK-type dehydrins, low molecular weight K-type dehydrin messengers become more abundant during the cold period, conferring protection to the plant against low temperatures and drought. Thus an increase in transcriptional activity of SK-type dehydrin genes during hardening is followed by a sharp increased in transcriptional activity of K-type dehydrin genes during the cold period where cold tolerance and dormancy is maximal. The corresponding dehydrin protein levels follow an analogous pattern. The invention exploits the positive correlation with the level and timing of induction of both SK and K-type dehydrins. The differences in transcriptional activity are used to determine a ratio between the SK-type and K-type dehydrins. This ratio may be determined once, to establish the level of stress tolerance in a plant, for instance after a (cold, dark and/or dry) period of hardening, or in a breeding program to select those plants in a group of plants that are most stress tolerant. The ratio may also be determined multiple times, preferably during fixed intervals, to be able to determine the timing and the level of induction of stress resistance, cold tolerance and dormancy in a plant. Absolute numbers for this ratio cannot be given, as they are of course dependent on the particular plant being tested, the particular dehydrins assayed and the sensitivity of the detection technique used. The difference in ratio between plants with relatively low and relatively high levels (compared to eachother) of SK-type and K-type dehydrins is indicative of the relative level of stress resistance in the plant. Similarly, observation of a relative shift in the ratio between SK- and K- type dehydrins in a single plant or variety of plants, shifting in time from an induction and relative abundance in SK- type dehydrins towards an induction and relative abundance of K- type dehydrins during acquiring of stress tolerance by a plant or plant variety is key to the current invention.

## Detailed description

In a first embodiment the current invention provides a method for determining stress tolerance in a plant, comprising the step of determining the ratio between an SK type dehydrin and a K type dehydrin, wherein the plant to be tested is a gymnosperm.

Stress tolerance in this specification is meant to comprise at least the stress factors induced by various field conditions such as drought, light conditions in terms of length and intensity, or low temperatures, as defined by temperatures under 10, 8, 6, 4, 2, 0, -2, -5 °C and below. Stress tolerance also comprises tolerance to drought and/or osmotic stress, which is known to be highly correlated to cold tolerance because of dessication of plants at low temperatures (Environmental Physiology of Plants. A.H. Fitter and R.K.M. Hay, Academic Press, 1987) and will for a large part determine survival and re-growth potential after a cold or dormancy period. A change in light regimen, which may be the result of a decrease in both artificial or natural day length or light intensity, results in a stress or dormancy response in a plant and subsequent hardening and induction of dormancy in a plant. The method according to the invention, determining the ratio between a K-type dehydrin and an SK-type dehydrin, may therefore be carried out once to determine the stress tolerance level in a plant, for instance after a period of hardening. The method may also be carried out multiple times under different temperature and/or daylight conditions to monitor the hardening response and induction of stress tolerance and/or dormancy in a plant. The time intervals of taking samples and determining the ratio may vary. Preferably at least 2, 3, 4, 5, or more samples are collected with intervals of 1, 2, 3, 4, 6 to 8 weeks. During hardening and induction of dormancy in a plant by exposure to a lowering of the temperature and/or a decrease in daylength or (cumulative) light intensity, the ratio between SK/K-type dehydrins will rise slowly (gradual induction of SK-type dehydrins), followed by a marked decrease in the ratio (induction of K-type dehydrins). When determining the stress tolerance of a group of plants, for instance for breeding purposes, those plants from a group of plants having the preferred ratio (having relatively high K-type dehydrins) may be selected. When determining the induction of stress tolerance in a plant by hardening during a season, the induction of hardening (decreased SK/K type ratio, relatively high K-type expression) may be monitored, for instance for agricultural and/or forestry purposes.

The method according to the invention is preferably carried out by detection of an an $SK_4$ type dehydrin and/or even more preferably an $SK_2$ type dehydrin. However, also other S-domain containing dehydrins with a varying number of K-segments may be suitably applied according to the invention. The K-type dehydrin may be any dehydrin not containing an S-domain and preferably not containing a Y-domain. The K-type dehydrin may contain one or more K-domains and preferably is a $K_2$ dehydrin, containing 2 K-domains. A K-type domain dehydrin does not comprise S- or Y-domains.

In a preferred embodiment, the method according to the invention is carried out detecting an $SK_2$ type dehydrin messenger RNA molecule having at least 85, 90, 95, 98 or 99 % identity with a Psdhn2 (SEQ ID No. 1), or a fragment, homolog or analog thereof, capable of hybridizing under stringent conditions.

In the same or another preferred embodiment the method according to the invention is carried out detecting a $K_2$ type dehydrin messenger RNA or cDNA has at least 85, 90, 95, 98 or 99 % identity Psdhn5 (SEQ ID No. 2), or a fragment, homolog or analog thereof, capable of hybridizing under stringent conditions.

The method according to the invention is carried out on a plant, wherein the plant to be tested for SK-type and K-type dehydrin ratio by mRNA expression or protein content is a gymnosperm, more preferably a gymnosperm of the family of Pinaceae. The invention is particularly suitable for testing of stress tolerance, in particular induction of hardiness, dormancy and/or cold tolerance for plants from the family Pinaceae, in particular from the genus Pinus, the genus Picea, the genus Pseudotsuga, the genus Tsuga, the genus Larix, the genus Abies and the genus Cedrus. Suitable species from these genuses comprise at least: from the genus Pinus in particular: *P. halepensis , P. nigra , P. pinaster , P. pinea , P. sylvestris , P. banksiana ,P. contorta, P. elliottii, P. flexilis , P. glabra , P. jeffreyi, P. lambertiana , P. ponderosa (syn. P. washoensis) , P. radiata, P. taeda, P. lawsonii, P. occidentalis, P. patula),* the genus Picea: *Picea abies, Picea glauca, Picea engelmannii, Picea sitchensis, Picea pungens)),* the genus Pseudotsuga: (*Pseudotsuga menziesii* (all subspecites), *Pseudotsuga lindleyana, Pseudotsuga macrocarpa, Pseudotsuga japonica, Pseudotsuga sinensis* (all subspecies)), the genus Larix: (*Larix decidua, Larix laricina, Larix occidentalis*) the genus Abies: (*Abies alba, Abies nordmanniana, Abies procera, Abies fraseri, Abies balsamea*), the genus Cedrus: (*Cedrus deodara, Cedrus libani*) and the genus Tsuga: (*T. canadensis, T. caroliniana, T. chinensis, T. diversifolia, T. dumosa, T. forrestii, T. heterophylla, T. mertensiana, T. sieboldii*).

The samples obtained from a plant for determining its content of SK-type and K-type dehydrin proteins and dehydrin messenger RNA's may be any part of the plant, such as leaves, flowers, roots, shoots, twigs, fruits, pollen, seeds, embryo's, seedlings, or tissue culture material such as single cells or callus. Particularly preferred is the use of buds, more in particular apical buds.

In a highly preferred embodiment the method according to the invention, the ratio of SK-type and K-type dehydrins is determined by quantitative PCR techniques on mRNA samples which have been converted to cDNA by a reverse transcriptase reaction (RT-PCR). Quantitative PCR may be carried out by conventional techniques and equipment, well known to the skilled person, described for instance in S.A. Bustin (Ed.), et aL, A-Z of Quantitative PCR, IUL Biotechnology series, no 5, 2005. Preferably, labeled primers or oligonucleotides are used to quantify the amount of reaction product. Other techniques capable of quantifying relative and absolute amounts of mRNA in a sample, such as NASBA, may also be suitably applied.

A convenient system for quantification is the immunolabeling of the primers, followed by an immuno-lateral flow system (NALFIA) on a pre-made strip (references: Kozwich et al., 2000, Koets et al., 2003 and van Amerongen et al., 2005), for the detection of the SK- and K-type amplification products. As an example, primers specifically capable of selectively amplying the SK-type dehydrin Psdhn2 and the K-type dehydrin Psdhn5 are given in table 3 in example 1. As a positive control for the RNA isolation, reverse transcriptase reaction, amplification reaction and detection step, amplification and detection of a constitutively expressed housekeeping gene may be included in the assay, such as ribosomal (18S) messenger RNA's, actin or GAPDH. Primers may be labeled with direct labels such as FITC (fluorescein), Texas Red, Rhodamine and others or with tags such as biotin, lexA or digoxigenin which may be visualized by a secondary reaction with a labeled streptavidin molecule (for instance with carbon or a fluorescent label) or a labeled antibody ( labeled with fluorescent molecules, enzymes, carbon, heavy metals, radioactive isotopes or with any other label).

In another embodiment, comparative hybridization is performed on mRNA or cDNA populations obtained from a plant or sample thereof, to one or more dehydrin type- specific sequences, which may optionally be tagged or labeled for detection purposes, or may be attached to a solid carrier such as a DNA array or microarray. Suitable methods for microarray detection and quantification are well described in the art and may for instance be found in: Applications of DNA Microarrays in Biology. R.B. Stoughton (2005) Annu.Rev.Biochem. 74:53-82, or in David Bowtell and Joseph Sambrook, DNA Microarrays: A Molecular Cloning Manual, Cold Spring Harbor laboratory press, 2003.

The invention also pertains to nucleic acid carriers, such as arrays and microarrays or DNA chips, comprising nucleotides on a glass, plastics, nitrocellulose or nylon sheets, silicon or any other solid surface, which are well known in the art and for instance described in Bowtell and Sambrook, 2004 (ibid) and in Ausubel et al., Current protocols in Molecular Biology, Wiley Interscience, 2004. A carrier according to the current invention comprises at least two (oligo-)nucleotide probes capable of selectively hybridizing with SK- type and K-type dehydrins. Preferably, the SK hybridizing (oligo-)nucleotide probe is or is a fragment derived of SEQ ID No. 1 and/or the K type hybridizing (oligo-) nucleotide probe is or is a fragment derived of SEQ ID No. 2.

In another embodiment, the method according to the invention and in particular the carrier comprising DNA fragments capable of selectively hybridizing to SK or K type dehydrins, may further comprise additional sequences that are known to be involved in stress or cold tolerance in plants. The current inventors have found the expression of newly identified sequences, SEQ ID No's 3 and 4, to be indicative of induction and/or increased levels of stress tolerance in plants. These sequences according to this invention have been found to be indicative for induction of cold tolerance and/or hardening and capable of increasing hardiness and cold tolerance, in particular in Pinaceae. The methods and the DNA carrier according to the invention may therefore further comprise an oligonucleotide sequence capable of selectively amplifying and/or hybridizing with sequences having at least 85, 90, 95, 98, 99 % identity with a sequence encoding *Pinus* cold tolerance related transcript (SEQ ID No. 3). This sequence is induced in stress or cold tolerant plants. The carrier may also further comprise an oligonucleotide sequence capable of hybridizing with sequences having at least 85, 90, 95, 98, 99% identity with a sequence encoding a *Pinus* alpha tubulin transcript (SEQ ID No. 4). The relative abundance of this sequence is decreased in cold or stress tolerant plants.

In another embodiment, the method according to the invention is carried out on samples obtained from a plant, whereby the ratio of SK-type and K-type dehydrins is determined by comparative analysis of protein samples obtained from the plant, for instance using mass spectrometry techniques, or preferably using dehydrin type specific antibodies. Such antibodies may optionally be labeled, tagged and/or attached to a (solid) carrier. Type specific antibodies for K-type and SK-type dehydrins are available in the art, Close,T.J., Fenton, R.D. and Moonan, F. (1993) Plant Mol.Biol. 23: 279-286 and available from, for instance; (Stressgen Biotechnologies Corp., Victoria, Canada). Suitable antibodies may also be generated by the skilled artisan. Generation of monoclonal or polyclonal antibodies capable of interacting with a specific protein or a (unique) fragment or sequence in that protein is a standard technique which can be found in many textbooks, such as Current Protocols in Immunology, Wiley Interscience, 2004.

The method according to the invention will be most usefull for a rapid stage determination of stress tolerance in nursery- and plantbreeding practice, in particular for Pinaceae and for forestry purposes. Determining the level of stress tolerance will be most suitable for testing Pinaceae seedlings in the autumn, before they are taken from the field and stored till spring in a climatised winter storage at approximately - 2 ˚C to + 4 ˚C. The level of hardening and hence the optimal timing for storage is determined by the method according to the invention. The ratios will be usefull as indicators for different stages of hardening or dormancy and the resulting stress tolerance, in particular frost tolerance and winter hardiness and re-growth potential in spring, for instance after winter storage.

The description also discloses methods and means for enhancing stress tolerance in a plant, preferably a gymnosperm plant and most preferably of the genus Pinaceae, the method comprising the step of providing to the plant a source of K-type dehydrins, in particular $K_2$ dehydrins, in order to decrease the ratio between SK-type and K-type dehydrins. Preferably the source is a gene encoding K-type dehydrins, the gene being operably linked with regulatory sequences capable of conferring expression and translation in the host plant. The gene may be comprised in an expression cassette in a vector, preferably an *Agrobacterium* vector or a viral vector known in the art of producing transgenic plants. Methods for transformation of Pinaceae using vectors or particle bombardment are for instance described in Tian et al., Planta, 213(6) 2001, Tang and Tian, J. Exp. Botany, vol 54 no. 383,2003, Aronen TS et al., Transgenic Res. 12 (3), 2003 and in Grant JE et al., Plant Cell reports, vol. 22 no. 12, 2004.

The promoter driving the expression of the K-type dehydrin may be a constitutive promoter, for instance a viral promoter (such as CaMV) or an inducible or regulatable promoter. Most preferably, the promoter is a temperature sensitive or temperature inducible promoter. Temperature dependent regulation of gene expression in plants is well described in the field and the selection and application of a temperature dependent promoter for a given plant of interest is well within the ability of a skilled artisan.

The description thus discloses a method to obtain transgenic plants, in particular transgenic Pinaceae, with an increased stress / cold tolerance and/or an accelerated and enhanced hardening, comprising a recombinant expression cassette comprising a promoter operably linked to a gene providing expression of a K-type dehydrin, preferably a gene having at least 85, 90, 95, 98, 99 % identity with a gene encoding a K2-type dehydrin from Pinus sylvestris, SEQ ID No. 2 (Psdhn5), SEQ ID No. 5 (Psdhn 3), SEQ ID No. 6 (Psdhn 4), SEQ ID No. 7 (Psdhn 6), SEQ ID No. 8 (Psdhn 7) and SEQ ID No. 9 (Psdhn 8).

In another aspect the present invention relates to a "kit" containing elements for use in the methods of the invention. Such a kit may comprise a carrier to receive therein one or more containers, such as tubes or vials. The kit may further comprise unlabeled or labeled oligonucleotide sequences of the invention, e.g. to be used as primers, probes, which may be contained in one or more of the containers, or present on a carrier. The oligonucleotides may be present in lyophilized form, or in an appropriate buffer. One or more enzymes or reagents for use in isolation of nucleic acids, purification, restriction, ligation and/or amplification reactions may be contained in one or more of the containers. The enzymes or reagents may be present alone or in admixture, and in lyophilised form or in appropriate buffers. The kit may also contain any other component necessary for carrying out the present invention, such as manuals, buffers, enzymes (such as preferably reverse transcriptase and a thermostable polymerase), pipettes, plates, nucleic acids (preferably labeled probes), nucleoside triphosphates, filter paper, gel materials, transfer materials, electrophoresis materials and visualization materials (preferably dyes, labeled antibodies or -enzymes) autoradiography supplies. Such other components for the kits of the invention are known per se.

**Definitions**

Hardening and tolerance to stress factors

In order to tolerate the stresses that they face, plants have to adapt their metabolism. Several cellular and metabolic functions are altered by low temperatures and freezing. Plant membranes undergo both qualitative and quantitative modifications during periods of cold acclimation and deacclimation. The lipid composition of the plasma membrane and chloroplast envelopes changes during cold acclimation. Carbohydrate content is known to vary according to the hardening status of a tissue. Starch concentrations decrease and the concentrations of soluble sugars increase in cold acclimating tissues of woody plants. The process of adaptation to low temperatures causes changes in the function of genes and proteins. The adaptation involves the modification of pre-existing proteins and the up- and down-regulation of gene expression or protein synthesis. Cold induced gene activity aids in the metabolic adjustment to low temperatures or confer freezing tolerance to tissues. In woody plants, many of the genes and proteins related to cold acclimation may also be connected to the dormancy status of the plants. Late Embryogenesis Abundant (LEA) proteins, in particular the D-11 family or group 2 LEA proteins, are called dehydrins. Dehydrin proteins are induced in plants by dehydration-related environmental stresses such as low temperature, drought or high salinity. These proteins, of variable molecular masses, have been found in many plant species. Indicative of dehydrins is the presence of one or several lysine-rich units called the K-segments conserved near the carboxy terminus of the protein and repeated several times throughout the sequence. Some dehydrins also possess a string of serine residues (S-segment). Another consensus sequence (DEYGNP), the Y-segment, can be found near the amino terminus of some of the dehydrins.

A DNA segment, in particular a segment containing a dehydrin encoding gene, is "operably linked" when it is placed into a functional relationship with another DNA segment. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

"Sequence identity" is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps). Preferred parameters for nucleic acid comparison include the following: Algorithm: Needleman and Wunsch, J. Mol.

Biol. 48:443-453 (1970); Comparison matrix: matches=+10, mismatch=0; Gap Penalty: 50; Gap Length Penalty: 3. Available as the Gap program from Genetics Computer Group, located in Madison, Wisconsin. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; a group of amino acids having acidic side chains is aspartic acid and glutamic acid and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

Nucleic acid sequences encoding dehydrin polypeptides and/or stress tolerance conferring activity may also be defined by their capability to hybridise with the (complementary strand of) the nucleotide sequence of SEQ ID No's: 1, 2, 5, 6, 7, 8, 9 or 10, preferably under moderate, or more preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65˚C in a solution comprising about 1 M salt, preferably 6 x SSC or any other solution having a comparable ionic strength, and washing at 65˚C in a solution comprising about 0,1 M salt, or less, preferably 0,2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity. Moderate conditions are herein defined as conditions that allow a nucleic acid sequences of at least 50 nucleotides, preferably of about 200 or more nucleotides, to hybridise at a temperature of about 45˚C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 99%.

## Figure Legends

Figure 1: Deduced amino acid sequences of the isolated dehydrins from *Pinus sylvestris.*

A. Both dehydrins, *Psdhn1* and *Psdhn2,* are compared with a dehydrin sequence of *Picea glauca, Pgdhn1.*
B. Comparison of the sequences from the low-molecular-weight dehydrins, *Psdhn3-8.* Conserved domains (Serine-stretch and K-segment) shared by all known dehydrins, are shown in bold. Dots indicate conserved amino acids in the actual comparison.
C. Consensus sequences for the different K-segments found in deduced amino acid sequences from the indicated pine species. For both *P.taeda* and *P.pinaster* Accession numbers are given.

Figure 2A and B: Characteristics of the physiological status of the one-year-old pine seedlings used for expression profiling. Freeze induced shoot electrolyte leakage data are presented for the provenances A70 (open squares) and Lindås (closed squares) together with the Cold Index (B panel, experienced hours below 5˚C). Standard errors of means are given.

Figure 3: Expression data of dehydrins in pine apical buds from both provenances, using the cDNA-microarray. Microarray data are 2log ratios calculated from fluorescence values of sample and reference probes of the indicated cDNA-clones. The right panel shows the RT-PCR data obtained from Lindås apical buds. Relative copynumbers were determined by extrapolation from a standard curve and the percentage of the maximal value is given. Standard deviations of duplicate measurements are indicated.

Figure 4: RT-PCR data of dehydrins in pine apical bud material.

A. $C_T$-values (means of duplicates), using the specific primer sets defined in Table 3, the 18S signal was used as the internal control.
B. Calculated fold change in expression relative to week 41 for A70 and to week 43 for Lindås, normalised using the 18S rRNA data. ($\Delta\Delta C_T = (C_{T,\ dehydrin} - C_{T,\ 18S})_{Time\ x} - (C_{T,\ dehydrin} - C_{T,18S})_{Time\ 0}$). Data are presented in the following order, *Psdhn1* (White), *Psdhn2* and *Psdhn5,* for each timepoint.
C. Hierachical clustering of the $C_T$-values of the three indicated dehydrins.

**Examples**

Materials and Methods

*Plant material for cloning purposes*

Apical bud material used for the construction of the cDNA Expression library was collected in week 48 of 1997 from two-year-old containerised *Pinus sylvestris,* grown outdoors in the Netherlands. The plants were obtained from Applied Plant Research (Boskoop, The Netherlands).

For construction of the subtraction libraries, apical buds from two-year-old *Pinus sylvestris* plants of the Dutch provenance Vosterbos were used. The containerised plants were obtained via a local grower and were grown outside. February the fifth (2001) apical buds were collected from 30 plants and frozen in liquid nitrogen (quiescent sample). At the same time another 30 plants were subjected to cold treatment during the night. Plants were put in a dark climate-controlled room where temperature decreased gradually from 20˚C to -10˚C. The plants were kept at -10˚C for five hours. Relative humidity varied between 80 and 90%. The next morning apical buds were collected from these plants and frozen in liquid nitrogen (cold treated sample). From the same batch of plants, thirty apical buds were collected and frozen in liquid nitrogen at the third of April 2001, shortly before bud-break (release sample).

*Plant material for expression analysis*

For expression analysis two provenances were used. A commercial British seedlot, A70 (55˚28'N to 57˚41'N) and seed from a Norwegian provenance, Lindås (60˚42'N; 5˚19'E). Both were sown on 14 May 2001 in HIKO 120-ss containers filled with peat (Pindstrup 2) and raised in a greenhouse in Aarslev, Denmark. During the greenhouse period the seedlings were irrigated regularly and liquid fertiliser (Pioner NPK Makro: 100 ppm N, 25 ppm P, 75 ppm P, 75 ppm K, 30 ppm Mg) was applied three times per week. On 10 August the seedlings were placed outside where they remained for the rest of the experimental period. After this date they were irrigated regularly but not fertilised. Every two weeks, starting in week 37 (10 September) and until week 4 (21 January), apical buds of 50 randomly selected seedlings were harvested and frozen in liquid nitrogen. At the same time shoot tip samples were tested for frost tolerance using freeze induced electrolyte leakage.

The Cold Index was determined by taking the air temperature ($T_{air}$) during the period from week 37 to week 4, as measured 0.5 m above the ground every half hour using a datalogger (Tinytalk, Gemini Dataloggers, Chichester, UK). A cold index (Ci ) accumulating the number of hours with $T_{air}$ < 5 ˚C was calculated from the temperature data.

*Freeze testing*

Shoot tip samples were tested for frost tolerance. On each date 30 randomly selected seedlings were sampled, and a 2-cm long stem segment (with needles) was excised from each seedling immediately below the apical bud. The segments were washed in tap water and subsequently rinsed in deionised water. Each sample was then placed in a 20-ml plastic bottle and capped. Half of the samples (15 replicates) were frozen in a programmable freezer at a rate of 2 ˚C h-1 to -15 ˚C. They were held at this temperature for 120 minutes before they were removed from the freezer and placed at - 1˚C overnight. The samples were taken out the following morning for assessment of frost injuries. The remaining 15 samples were held at +2˚C as unfrozen control until freeze treatments had ended.

For the evaluation of frost injury, shoot electrolyte leakage of the frost treated samples and unfrozen controls was assessed using the conductivity method modified from McKay (1992). After rewarming the samples to room temperature, 20 ml of deionised water with low electrical conductivity (C0) was added to each bottle. The samples were subsequently incubated in darkness at room temperature for 24 hours ± 15 min. After inverting the incubated samples five times, the conductivity of the water (Cl) was measured with a conductivity meter. The samples were then autoclaved at 120 ˚C and 1.2 bar for 60 minutes to kill the cells. After cooling the samples to room temperature, a second recording of conductivity (C2) was made. The shoot electrolyte leakage (SEL) was calculated as relative conductivity (RC):

$$RC = \frac{C_1 - C_0}{C_2 - C_0} \cdot 100\%$$

The electrolyte leakage caused by freezing was expressed as the difference between RC of a frozen sample and RC of a corresponding control sample and designated SELdiff-15. A decline in SELdiff with time would then indicate that the shoot tissue had become more frost tolerant to -15 ˚C. (Lindström & Håkansson 1996, Stattin et al. 2000).

*cDNA library construction*

RNA was isolated from frozen buds according to Chang et al. (1993). mRNA was extracted using the Dynabeads mRNA Purification Kit (Dynal Biotech, Germany) and used for the construction of the cDNA libraries. The cDNA Expression library (EL) was made using the λ ZAP II system (Stratagene, The Netherlands) following manufacturers instructions.

Subtraction libraries were made using the PCR Select cDNA Subtraction Kit (BD Biosciences Clontech, Europe) according to manufacturers' instructions. Two different libraries were made in order to enrich for genes that were either specifically expressed during quiescence or as a result of the applied short-term low temperature treatment. In order to enrich for quiescence related genes, RNA from the quiescent sample was used as tester whereas a mixture of equal portions of RNA from the cold treated and the release samples was used as driver resulting in quiescent enriched library (QL). For the enrichment of the cold induced genes, RNA from the cold treated sample was used as tester whereas a mixture of equal portions of RNA from the quiescent and the release sample was used as driver resulting in cold enriched library (CL). The Advantage PCR Kit (BD Biosciences Clontech) was used for the amplification of the libraries. PCR conditions were according to the manufacturer protocol. The products, ranging in size from three hundred up to thousand basepairs, were cloned in the pGEM-T Easy Vector (Promega, The Netherlands).

*Isolation of dehydrin cDNA clones*

From the Expression library $1.10^5$ plaques were screened using the picoBlue Immunoscreening Kit (Stratagene, The Netherlands) and the Anti-Dehydrin polyclonal antibody from Stressgen (Canada). Positive plaques were isolated and the vector (pBluescript $SK^-$) containing the putative dehydrin cDNA was excised following the protocol of Stratagene. The clones were sequenced and identified using BLAST available on the web.

Besides, randomly selected clones from the Expression library (1000 clones) and both the Subtraction libraries (500 clones each) were sequenced and identified using BLAST. Contig analysis of dehydrin related clone sequences was done using DNASIS Ver. 2.6 (Hitachi Software Engineering Co.).

In order to isolate missing parts at the 3'- or 5'- ends of the clones, RACE reactions were performed. The GeneRacer Kit, purchased from Invitrogen (Life Technologies, The Netherlands), was used. Pooled RNA from the whole range of samples taken from the one-year-old Lindås seedlings was used to make cDNA following manufacturers' instructions.

*Expression analysis using the microarray*

A microarray was prepared using 1500 clones selected from all three cDNA libraries. A selection of the identified dehydrins was spotted on the microarray. To monitor gene expression during autumn and winter, a Cy5-labeled cDNA population was prepared from each of the samples taken from the two mentioned provenances. For each hybridisation, a single Cy3-labeled cDNA population, prepared from unrelated Scots pine bud material collected in the Netherlands, was used as a common reference. Hybridisation procedures were as described by Aharoni et al. (2000) and van Doom et al. (2004). Relative expression of each individual clone is presented as the 2 log ratio calculated from the fluorescence value of sample and reference probes.

*RT-PCR measurements*

In order to verify the data obtained from the microarray, Realtime RT-PCR was used. Total RNA was isolated as described before and RNA preparations were treated with DNAseI (AP Biotech) and purified using the RNeasy system (Qiagen, Westburg, The Netherlands). Half a microgram of pure total RNA was used for the synthesis of cDNA. Anchored oligo $(dT)_{23}$ from SIGMA (The Netherlands) was used in combination with Superscript II Reverse Transcriptase from Invitrogen (Life Technologies, The Netherlands). Dilutions of this cDNA were used for Realtime PCR using the qPCR Core Kit (Eurogentec, Belgium) and the iCycler system (BIORAD Laboratories, The Netherlands). Primer sets used are described in Table 3. The annealing temperature was chosen in such a way that no by-products were being produced, as judged from melt-curve analysis and agarose gel-electrophoresis. PCR efficiencies were between 95 and 110% measured by using serial dilutions of pure target. PCR product formation was detected using Sybr Green I intercalation. A threshold was set, which intersected the amplification curves in the linear region of the semi-log plot. The point at which the curve crosses the threshold, the so-called threshold value ($C_T$), was taken as a measure for the relative amount of target present in a certain sample. Relative changes in gene expression were calculated using the $2^{-\Delta\Delta C_T}$ method described by Livak and Schmittgen (2001). The signal obtained from a PCR on the same batch of pine cDNA using primers homologous to *Arabidopsis* 18S rRNA, was taken as the reference for normalisation. The sequences of the 18S rRNA primers were as follows, 5'TGACGGAGAATTAGGGTTCG and 5'CCTCCAATGGATCCTCGTTA. The expected size of the PCR fragment was 195 base pairs, based on sequence information from the *Arabidopsis* sequence. Hierarchical clustering of realtime RT-PCR data was performed using Acuity 3.1 software (Axon Instruments Inc.).

## Results

Example 1

*Introduction*

*Pinus sylvestris* (Scots pine) seedlings display a strictly periodic growth pattern over the year. When seedlings are placed under different light regimes, growth cessation and bud-set always takes place (Christersson 1978, Ekberg et al. 1979). Growth cessation and frost tolerance develop in parallel (Dormiling 1993) and are triggered by both short days and low temperature, especially in one-year-old seedlings (Christersson 1978, Repo et al. 2000).

For seedlings of *Pinus sylvestris* different stages of hardening have been reported. As growth ceases the bud enters a stage of ecodormancy or quiescence (Dormling 1993, Lang 1987). This stage is characterised by intermediate frost tolerance levels. The timing of this first stage varies among pines originating from different latitudes. Growth ceases earlier in northern provenances compared to southern provenances (Repo et al. 2000). This seems to be directly influenced by differences in photoperiod (Ekberg 1979). As hardening proceeds, a second stage is entered in which buds develop rest (endodormancy, Lang 1987) and frost tolerance is at its maximal level (Dormling 1993). Variation in rates of hardening was found between provenances from different latitudes (Repo et al. 2000). The final level of frost tolerance seems to be influenced primarily by the actual cold sum and not so much by latitudinal origin.

Seasonal variation in dehydrin protein content has been studied in different tissues of Scots pine (*Pinus sylvestris*) and several different dehydrins have been identified using a K-segment specific antibody (Close et al. 1993, Kontunen-Soppela and Laine 2001). It was found that dehydrin proteins are present throughout the year but levels vary in a tissue specific manner. In bud tissue a 60 kDa dehydrin is present at high levels during the winter.

This specification provides the cDNA-cloning of several dehydrins, representing different classes, from dormant apical buds of Scots pine. The expression profile of a selection of these genes was studied during autumn and winter in two provenances from different latitudes. The results indicate distinct roles for the different dehydrin types K and SK in the protection of pine buds during this period. The changes in gene expression, SK-/K-type ratio and the differences in timing of those changes between the two provenances in relation to frost tolerance are exploited to determine cold tolerance and conferring cold tolerance to a plant.

*Identification of dehydrin cDNA clones*

Immunoscreening of the EL cDNA library resulted in the isolation of four clones that show high homology with known dehydrin sequences present in the public databases. Based on sequence similarity with a dehydrin from white spruce, *Pgdhn1* (Acc.no. AF1009916), one of the clones, designated *Psdhn1,* was probably full-length. Since the deduced amino acid sequence contained an eight-serine stretch and four conserved repeats of the lysine-rich K-segment (Table 1 and Figure 1A, SEQ ID No. 10), it could be classified as a $SK_4$ type dehydrin. The amino acid sequence displayed homology with sequences derived from the genomic fragments isolated by Mikkonen (Acc.no. AF359133 and AF359134). Both deduced amino acid sequences contain an S-segment and four K-segments, like *Psdhn1.* Predictions with regard to (post translational) modifications were made using the PredictProtein server (http://cubic.bioc.columbia.edu/predictprotein/predictprotein.html). Results are presented in Table 2. In the case of *Psdhn1* several possible locations for phosphorylation were found and one putative myristoylation site. Additionally, a nuclear localisation signal could be recognised.

Table 1. Characteristics of the dehydrin cDNA-clones isolated from the libraries. Besides the total number of clones that represent each dehydrin, the cDNA-library from which each individual originates, is indicated. Values for the Molecular Weights (MW) and the Isoelectric Point (pI) are calculated from deduced amino acid sequences.

| cDNA-clone | total number of cDNA clones | number of cDNA clones per library | | | full-length | MW (kD) | pI | conserved domains | | Acc.no. |
|---|---|---|---|---|---|---|---|---|---|---|
| | | expression library | enriched quiescent | enriched cold | | | | S | K | |
| *Psdhn1* | 1 | 1 | | | yes | 26 | 10.2 | 1 | 4 | AJ289610 |
| *Psdhn2* | 1 | 1 | | | yes | 14 | 6.9 | 1 | 2 | AJ512361 |
| *Psdhn3* | 8 | 6 | 2 | | yes | 10 | 8.4 | - | 2 | AJ512362 |
| *Psdhn4* | 2 | | 2 | | yes | 10 | 8.9 | - | 2 | AJ512363 |
| *Psdhn5* | 20 | 11 | 7 | 2 | yes | 10 | 7.9 | | 2 | AJ512364 |
| *Psdhn6* | 1 | 1 | | | yes | 11 | 9.0 | - | 2 | AJ512365 |
| *Psdhn7* | 8 | 7 | 1 | | yes | 10 | 7.7 | - | 2 | AJ512366 |
| *Psdhn8* | 1 | | 1 | | no | | | - | 2 | AJ512367 |

Table 2. Motifs found in the protein sequences of a selection of the cloned pine dehydrins. Predictions are made using the Predict Protein facility available on the web (Columbia University Bioinformatics Centre). Amino acid positions are given, number of motifs between brackets.

| dehydrin type | | amidation myristoylation glycosylation phosphorylation other | | | | |
|---|---|---|---|---|---|---|
| *Psdhn1* | $SK_4$ | | 91(1) | | 32,39,58,83,98, 104,107,209 (8) | Nuclear Localization Signal; GREKKKKKQKWRKRR |
| *Psdhn2* | $SK_2$ | 17,95,102 (3) | 51(1) | 82 (1) | 75,88,106,116 (4) | |
| *Psdhn7* | $K_2$ | 14,35 (2) | 65(1) | 90 (1) | 92 (1) | Putative disulfide bonding, (position 25) |

Two cDNA clones showed homology with other dormancy- and desiccation-related proteins. Although the signals were less intense than the signals of the dehydrin homologues, they could be easily recognised. One of the clones was related to DRM1 from *Pisum sativum* (Acc.no. AF029242) and the other one was similar to clone PCC 13-62 from *Craterostigma planagineum* (Acc.no. P22242). Both cDNA-sequences encode for lysine rich proteins but they do not contain the K-segment specific consensus sequence.

From the 2000 sequenced clones, randomly selected from the three cDNA-libraries, 5% were identified as dehydrin homologues. Fifty of them were picked from the Expression library, forty-five from the library enriched for quiescent genes and 7 from the library enriched for low temperature induced genes. Contig analysis revealed 7 additional distinct dehydrin genes, designated *Psdhn2* through *Psdhn8* (Table 1 and Figure 1). High numbers *of Psdhn3, Psdhn5* and *Psdhn7* were picked (Table 1).

Based on sequence similarities it was concluded that part of the *Psdhn2* (SEQ ID No. 1) and *Psdhn8* (SEQ ID No. 9) sequence information was missing. However, *Psdhn8* appeared to be almost full length whereas *Psdhn2* was most probably missing a considerable part of the 5'end. So therefore we focused on getting additional sequence information only for *Psdhn2.* The primer that was chosen for this purpose was similar to the one used for RT-PCR (Table 3). The deduced amino acid sequence of the extra fragment was 56 residues long and had high homology with the dehydrin from white spruce, *Pgdhn1* (Figure 1A). *Psdhn2* was classified as an $SK_2$-type dehydrin. The consensus sequence of the K-segments present in both *Psdhn1* and *Psdhn2* was much alike the sequences found in several dehydrins of different pine species (Figure IC). *Psdhn2* showed high homology, 72 percent at the nucleotide level, with all published dehydrin sequences from *Pinus pinaster.*

Table 3. Primer sequences used for RT-PCR and RACE reactions. Primerset Psdhn3-8 detects all mRNA molecules from the corresponding genes simultaneously.

| Target | Primersequence | Orientation | appliedT$_{anneal}$ (˚C) | Productsize (bp) | Position |
|---|---|---|---|---|---|
| Psdhn1 | 5'CACACGGGTTTGATAGG | Forward | 55 | 171 | within coding region |
| Psdhn1 | 5'TGATCTGAAGAATGCTGTCC | Reverse | | | |
| Psdhn2 | 5'TGAGAATAATGGTACGTGCGT GTTG | Forward | 55 | 156 | 3'untranslated region |
| Psdhn2 | 5'CTGAAGGTAAGCTCGTACCGA AACC | Reverse | | | |
| Psdhn5 | 5'GTTCAGGGCATTGCTTAGGAG | Forward | 55 | 129 | 3'untranslated region |
| Psdhn5 | 5'GCAAATACCGACCTCACCATC | Reverse | | | |
| Psdhn3-8 | 5'GGAAGAAACCGGGAATGG | Forward | 55 | 163 | within coding region |
| Psdhn3-8 | 5'TTTTGTTGTCCCGGCAGC | Reverse | | | |

*Psdhn3-8,* represented by high numbers of clones mainly present in both the Expression library and the library enriched for quiescent cDNA clones, displayed a high degree of homology (Figure 1B). They all contained two K-segments so they were classified as $K_2$-types. Based on 5'RACE reactions, using the group-specific primer (Table 3), these clones were judged to be full-length. A specific EST from *Pinus taeda* (BG040304) displayed high homology, 75 percent at the nucleotide level, with this group of dehydrins. The 3'K-segment consensus sequence appeared to be more conserved between the species (Figure 1C) than did the 5'K-segment. The consensus sequence is clearly more extensive than the consensus sequences of the K-segment found in *Psdhn1* and *Psdhn2.*

Predictions with regard to putative protein modifications of *Psdhn2-8* were made using the PredictProtein server and the results are shown in Table 2 (*Psdhn7* being the representative of the whole group of $K_2$-type dehydrins). Several kinds of modifications are possible and the only difference between *Psdhn2* (SEQ ID No. 1) and *Psdhn3-8* is that the latter ones are predicted to form disulfide bonds. The amino acid sequences of PSDHN 1 - 8 proteins can be found in SEQ ID No's 11 to 18 respectively.

Example 2, *Characteristics of the physiological status of the pine seedlings used for transcriptional profiling*

Results of frost tolerance measurement of apical shoots for both pine provenances, A70 and Lindås are shown in Figure 2. At week 39 the SEL diff-15 value was found to be higher in A70 compared to Lindås. From week 43 on, there is no significant difference between the two provenances examined. Both remained frost tolerant for the rest of the experimental period. Comparison of the frost tolerance data with the Cold Index (experienced hours below 5˚C) showed that the shoots have reached their maximal level of frost tolerance prior to a sharp increase in experienced cold.

Expression of dehydrins in pine apical buds was monitored from week 39 2001 until week 04 2002. Results from the microarray hybridisations are presented in Figure 3. Data from *Psdhn1* and that of a highly homologous clone, 95% at the nucleotide level, were taken together and the same holds for *Psdhn2,* in this case the homology between the two clones was 85%, enough to get cross-hybridisation. Eleven clones on the microarray represent dehydrins *Psdhn3-8* and their expression profiles were taken together in the same figure.

Three general expression profiles could be distinguished for the three structurally different classes described in this report. *Psdhn1* expression showed minor fluctuations. However slightly elevated mRNA levels could be observed in the middle of the experimental period for both provenances. *Psdhn2* mRNA levels decreased over time. Apart from an apparent dip in RNA levels at week 41, levels remained relatively constant and then dropped rapidly in the course of 4 weeks. Differences in timing of this drop were observed between the two provenances, which occurred earlier in apical buds of Lindås (from week 45 on) than in A70 (from week 49 on).

The opposite was found for the large group of dehydrins, *Psdhn3-8.* The mRNA levels of this group increased during the experimental period. In apical buds of A70 a sharp increase is observed in week 43 and in Lindås in week 45. The increment in mRNA levels of this group of genes is larger for A70 compared to Lindås. During the rest of the experimental period these mRNA levels remained high and relatively constant.

Microarray and RT-PCR results were in agreement. RT-PCR data for Lindås were shown in Figure 3 and additional timepoints were included in order to match the dataset obtained for A70. In the RT-PCR, primers were used which amplify all six members of the *Psdhn3-8* group simultaneously in order to maintain the same level of specificity as in microarray hybridisations.

In Figure 4 RT-PCR data are shown for *Psdhn1* (SEQ ID No. 10), *Psdhn2* (SEQ ID No. 1) and *Psdhn5* (SEQ ID No. 2). The points are chosen in such a way as to represent the different stages with respect to the expression profiles of the different dehydrins. In this case a specific primer set for *Psdhn5* was used. Relatively high numbers of cDNA clones were present for this gene in the cDNA libraries (Table 1).

High $C_T$-values were measured for both provenances using *Psdhn1* primers reflecting the relative low abundance of the corresponding mRNA during the whole experimental period. In stage I, higher $C_T$-values for *Psdhn5* were found for provenance A70 compared to Lindås, which points to lower mRNA levels. The difference between the provenances disappeared after the rise in mRNA leves in stage II. Low $C_T$-values found with *Psdhn5* primers in this stage indicate high mRNA levels. Figure 4B shows that within stage II one can discriminate between early (IIA) and late (IIB) stages. *Psdhn2* contributes mainly to that difference. Dehydrin $C_T$-values were used for hierarchical clustering and the result of it is shown in Figure 4C. This visualises clearly the different stages.

In conclusion, differential expression of structurally divergent dehydrins as shown in this study indicates specialised functions during autumn and winter in apical buds. Although frost tolerance and dormancy develop simultaneously in *Pinus sylvestris* seedlings, frost tolerance seems to be the most consistent feature. Growth cessation always takes place but true rest (endodormancy in the terminology of Lang 1987) is not always reached (Dormling 1993). Dormancy, measured by monitoring days-to-bud-break of seedlings transferred at various timepoints to optimal growing conditions, did not show significant variation during the period examined. The results from the frost tolerance measurements however, are clear (Figure 2). It is known from studies using plants from different origins that northern provenances develop frost tolerance earlier than plants from southern origins (Dormling 1993, Repo et al. 2000) even when grown under identical conditions (Perks and McKay 1997). Latitudinal origin accounts for the lower freeze induced Shoot Electrolyte Values (higher frost tolerance) at week 39 for provenance Lindås. The fact that we examined one-year-old seedlings, which haven't experienced a winter before, points to a heritable trait. Comparison of the development of frost tolerance with the experienced cold, measured as the Cold Index (Figure 2), indicates that hardening is complete before a sharp rise in Cold Index. This again confirms that hardening is a highly regulated process that is triggered by genetic and environmental factors exerting their influence before real harsh conditions occur.

During the process of hardening, different structural types of dehydrin play different roles. As was already reported by Kontunen and Laine (2001), dehydrin protein can be detected throughout the season but certain dehydrin proteins were mainly detected during the growing season or during winter.

The current inventors established that during autumn and winter the mRNA level of $SK_4$ type *Psdhn1* changes to some extent. This dehydrin may contribute to a certain base level of resistance to dehydration or has a function in protecting specific cellular structures. Abundance of $SK_2$ type *Psdhn2* however is high in autumn, when hardening develops, and decreased during winter. A large group of $K_2$ type dehydrin genes, *Psdhn3-8,* showed increasing mRNA levels in autumn, before maximal levels of frost tolerance were reached. Realtime RT-PCR was used to obtain data specific for all dehydrin types and in figure 4 data are presented for *Psdhn5.* The expression profile of this dehydrin gene matched the profile obtained from the microarray.

In summary, high expression of the $SK_2$ type *Psdhn2* is found when hardening proceeds. Subsequently, increase of $K_2$ type *Psdhn3-8* mRNA levels coincide with high levels of frost tolerance. In comparison with Lindås, the rise in *Psdhn3-8* mRNAs found in A70 is earlier and the increment is larger (Figure 3). This group of dehydrins contributes to high levels of frost tolerance, resulting from the fact that A70 needs a steeper rise in frost tolerance to reach the same level as Lindås at week 43. Low initial mRNA levels of *Psdhn3-8* are associated with the low level of frost tolerance found in shoots of A70 pines. The most indicative parameter for induction of hardening, dormancy and in particular stress and/or cold tolerance is therefore the ratio between SK and K type dehydrins, shifting from high $SK_4$, $SK_2$ and relatively low $K_2$, to a lower $SK_4$, $SK_2$ and a higher $K_2$ level.

Hence, with respect to dehydrin expression, different phases can be recognised during autumn and winter. This is evidenced by exploring various calculation methods using RT-PCR data. The results of this are shown in Figure 4. These data show that the expression pattern of the specified dehydrin $Psdhn_5$, equals that of the general pattern of *Psdhn 3, 4, 6, 7, 8* (SEQ ID No's 5, 6, 7, 8 and 9 respectively) found using the microarray.

The clearest distinction between the phases can be obtained using hierarchical clustering. Here it is evident that even the minor variation in expression of the $SK_4$ *Psdhn1* gene does contribute to the stage definition. When these figures are combined with the frost tolerance data from Figure 2, a general observation is that when stage II is entered, frost tolerance is reaching its maximal value soon. When this pattern is established for different provenances or different offspring in a crossing/breeding experiment, this set of indicator genes may be applied according to the method of this invention to calculate a ratio between SK-type and K-type dehydrins. This will be most usefull for a rapid stage determination in nursery- and plantbreeding practice, in particular for Pinaceae and for forestry purposes. Determining the level of stress tolerance will also be most suitable for testing Pinaceae seedlings in the autumn, before they are taken from the field and stored till spring in a climatised winter storage at approximately - 2 °C to + 4 °C. The level of hardening and hence the optimal timing for storage is determined by the method according to the invention. The ratios will be usefull as indicators for different stages of hardening or dormancy and the resulting stress tolerance, in particular frost tolerance and winter hardiness and re-growth potential in spring, for instance after winter storage.

**References:**

Aharoni, A., L.C. Keizer, H.J. Bouwmeester, Z. Sun, M. Alvarez Huerta, H.A. Verhoeven, J. Blaas, A.M. van Houwelingen, R.C. de Vos, H. van der Voet, R.C. Jansen, M. Guis, J. Mol, R.W. Davis, M. Schena, A.J. van Tunen, A.P. O'Connell. 2000. Identification of the SAAT gene involved in strawberry flavor biogenesis by use of DNA microarrays. Plant Cell 12(5):647-662.

Aart van Amerongen and Marjo Koets (2005) Simple and rapid bacterial protein and DNA diagnostic methods based on signal generation with colloidal carbon particles. In: Rapid methods for biological and chemical contaminants in food and feed. Eds. A. van Amerongen, D. Barug and M. Lauwaars, Wageningen Academic Publishers, Wageningen, The Netherlands, ISBN: 9076998531, pages 105-126.

Alsheikh, M.K., B.J. Heyen and S.K. Randall.2003. Ion binding properties of the dehydrin ERD14 are dependent upon phosphorylation. J.Biol.Chem.278(42):40882-40889.

Artlip, T.S., A.M. Callahan, C.L. Basset and M.E. Wisniewski. 1997. Seasonal expression of a dehydrin gene in sibling deciduous and evergreen genotypes of peach (Prunus persica (L.) Batsch). Plant Mol. Biol. 33:61-70.

Bigras, F.J. 1996. Conifer bud dormancy and stress resistance: A forestry perspective. In Plant dormancy. Physiology, Biochemistry and Molecular Biology. Ed. G.A. Lang. CAB International, Oxon UK, pp 171-192.

Campbell, S.A. and T.J. Close. 1997. Dehydrins: genes, proteins, and associations with phenotypic traits. New Phytol. 137:61-74.

Chang, S., J. Puryear and J. Cairney. 1993. A simple and efficient method for isolating RNA from pine trees. Plant Mol. Biol. Rep. 11(2):113-116.

Choi, D.W., B. Zhu and T.J. Close. 1999. The barley (Hordeum vulgare L.) dehydrin multigene family: sequences, allele types, chromosome assignments, and expression characteristics of 11 Dhn genes of cv Dicktoo. Theor. Appl. Genet. 98:1234-1247.

Christersson, L. 1978. The influence of photoperiod and temperature on the development of frost hardiness in seedlings of Pinus sylvestris and Picea abies. Physiol.Plant. 44:288-294.

Close, T.J., R.D. Fenton and F. Moonan. 1993. A view of plant dehydrins using antibodies specific to the carboxy terminal peptide. Plant Mol. Biol. 23:279-286.

Close, T.J. 1996. Dehydrins: Emergence of a biochemical role of a family of plant dehydration proteins. Phys. Plant. 97:795-803.

Close, T.J. 1997. Dehydrins: A commonalty in the response of plant to dehydration and low temperature. Phys. Plant. 100:291-296.

Doom, W.G., P.A. Balk, A.M. van Houwelingen, F.A. Hoeberichts, R.D. Hall, O. Vorst, C. van der Schoot and M.F. van Wordragen. 2003. Gene expression during anthesis and senescence in Iris flowers. Plant.Mol.Biol. 53:845-863.

Dormling I. 1993. Bud dormancy, frost hardiness, and frost drought in seedlings of Pinus sylvestris and Picea abies. In Advances in plant cold hardiness. Ed. Paul H. Li and Lars Christersson. CRC Press, Florida, pp 285-298.

Ekberg I., G. Eriksson and I. Dormling. 1979. Photoperiodic reactions in conifer species. Holarctic Ecology 2:255-263.

Heyen. B.J., M.K. Alsheikh, E.A. Smith, C.F. Torvik, D.F. Seals and S.K. Randall. 2002. The Calcium-binding activity of a vacuole-associated, dehydrin-like protein is regulated by phosphorylation. Plant Physiol. 130:675-687.

Jarvis, S.B., M.A. Taylor, M.R. Macleod and H.V. Davies. 1996. Cloning and characterisation of the cDNA clones of three genes that are differentially expressed during dormancy-breakage in the seeds of Douglas fir (Pseudotsuga menzeisii). J. Plant Physiol. 147:559-566.

Karlson D.T., T. Fujino, S. Kimura, K. Baba, T. Itoh and E.N. Ashworth. 2003. Novel plasmodesmata association of dehydrin-like proteins in cold acclimated red-osier dogwood (Cornus sericea). Tree Physiol. 23 :759-767.

Koag, M., R.D. Fenton, S. Wilkens and T.J. Close. 2003. The binding of Maize DHN1 to lipid vesicles. Gain of structure and lipid specificity. Plant Physiol. 131 :309-316.

Marjo Koets, Nathalie Barbier, Emil Wolbert, Hans Mooibroek and Aart van Amerongen (2003) Rapid and simple one-step and mini-array methods to detect and quantify amplified DNA and proteins using ligand-labeled colloidal particles. In: Proceedings EURO FOOD CHEM XII - Strategies for Safe Food, 24-26 September 2003, Brugge, Belgium, pages 121-124.

Kontunen-Soppela, S. and K. Laine. 2001. Seasonal fluctuation of dehydrins is related to osmotic status in Scots pine needles. Trees 15:425-430.

Diane Kozwich, Kristine A. Johansen, Keli Landau, Christopher A. Roehl, Sam Woronoff, and Patrick A. Roehl (2000) Development of a novel, rapid integrated Cryptosporidium parvum detection assay, Applied and Environmental Microbiology 66, 2711-2717. (*Xtrana Inc., Denver, Colorado 80230*)
Lang, G.A. 1987. Dormancy: a new universal terminology. HortScience 22:817-820. Levi, A., G.R. Panta, C.M. Parmentier, M.M. Muthalif, R. Arora, S. Shanker and L.J. Rowland. 1999. Complementary DNA cloning, sequencing and expression of an unusual dehydrin from blueberry floral buds. Physiol.Plant. 107:98-109
Lim, C.C., S.L. Krebs and R. Arora. 1999. A 25-kDa dehydrin associated with genotype- and age-dependent leaf freezing-tolerance in Rhododendron: a genetic marker for cold hardiness? Theor. Appl. Genet. 99:912-920.
Lindström, A. and L. Häkansson. 1996. EC-metoden - et sätt at bestämma skogplantors lagringsbarhet. Sveriges Lantbruksuniversitet, Institutionen for skogsproduktion. Stencil nr. 95, 30 pp.
Livak K.J. and T.D. Schmittgen. 2001. Analysis of relative gene expression data using real-time quantitative PCR and the 2-ΔΔCT method. Methods 25:402-408.
Momma, M., S. Kaneko, K. Haraguchi and U. Matsukura.(2003) Peptide mapping and assessment of cryoprotective activity of 26/27-kDa dehydrin from soybean seeds. Biosci.Biotechnol.Biochem.67(8):1832-1835.
McKay, H.M. 1992. Electrolyte leakage from fine roots of conifer seedlings: a rapid index of plant vitality following cold storage. Can.J.For.Res. 22: 1371-1377.
Nylander, M., J. Svensson, E.T. Palva and B.V. Welin. 2001. Stress-induced accumulation and tissue-specific localization of dehydrins in Arabidopsis thaliana. Plant Mol. Biol. 45:263-279.
Perks, M.P. and H.M. Mckay. 1997. Morphological and physiological differences in Scots pine seedlings of six different origins. Forestry 70(3):223-232.
Porat, R., D. Pavoncello, S. Lurie and T.G. Mccollum. 2002. Identification of a grapefruit cDNA belonging to a unique class of citrus dehydrins and characterization of its expression patterns under temperature stress conditions. Physiol.Plant. 115:598-603.
Repo, T., G. Zhang, A. Ryyppö, R. Rikala and M. Vuorinen. 2000. The relation between growth cessation and frost hardening in Scots pins of different origins. Trees 14:456-464.
Richard, S., M. Morency, C. Drevet, L. Jouanin and A. Seguin. 2000. Isolation and characterization of a dehydrin gene from white spruce induced upon wounding, drought and cold stress. Plant Mol. Biol. 43:1-10.
Rinne, P.L.H., P.L.M. Kaikuranta, L.H.W. van der Plas and C. van der Schoot. 1999. Dehydrins in cold-acclimated apices of birch (Betula pubescens Ehrh.): production, localization and potential role in rescuing enzyme function during dehydration. Planta 209:377-388.
Rowland L.J. and R. Arora. 1997. Proteins related to endodormancy (rest) in woody perennials. Plant Science 126: 119-144.
Stattin, E., Hellqvist, C. and A. Lindström. 2000. Storability and root freezing tolerance of Norway spruce (Picea abies) seedlings. Can.J.For.Res. 30:964-970.
Wang, W., D. Pelah, T. Alergand, O. Shoseyov and A. Altman. 2002. Characterization of SP1, a stress-responsive, boiling soluble, homo-oligomeric protein from aspen. Plant Physiology 130:865-875.
Welling, A., T. Moritz, E.T. Palva and O. Junttila. 2002. Independent activation of cold acclimation by low temperature and short photoperiod in hybrid aspen. Plant Physiology 129:1633-1641.
Welling, A., P. Rinne, A. Viherä-Aarnio, S. Kontunen-Soppela, P. Heino and E.T. Palva. 2004. Photoperiod and temperature differentially regulate the expression of two dehydrin genes during overwintering of birch (Betula pubescens Ehrh.). J.Exp.Botany 55 (396):507-516.
Wise, M.J., A. Tunnacliffe. 2004. POPP the question: what do LEA proteins do? Trends in Plant Science 9(1): 13-17.
Zhu, B., D.W. Choi, R. Fenton and T.J. Close. 2000. Expression of the barley dehydrin multigene family and the development of freezing tolerance. Mol.Gen.Genet. 264:145-153

**Claims**

1. A method for determining stress tolerance in a plant, comprising the step of determining the ratio between an SK-type dehydrin and a K-type dehydrin, wherein the plant to be tested is a gymnosperm.

2. The method according to claim 1 wherein the ratio is determined multiple times under different field conditions.

3. The method according to claim 1 wherein the SK type dehydrin is an $SK_4$ dehydrin or preferably an $SK_2$ dehydrin, and the K type dehydrin is a $K_2$ dehydrin.

4. The method according to any of the preceding claims wherein the $SK_2$ type dehydrin has at least 85% identity with Psdhn2 (SEQ ID NO: 1).

5. The method according to any of the preceding claims wherein the $K_2$ type dehydrin has at least 85% identity with Psdhn5 (SEQ ID NO: 2).

6. The method according to any of the preceding claims, wherein the plant to be tested is of the family Pinaceae.

7. The method according to any of the preceding claims wherein the plant to be tested is selected from the genus Pinus, the genus Picea, the genus Pseudotsuga, the genus Tsuga, the genus Larix, the genus Abies and the genus Cedrus.

8. The method according to any of the preceding claims wherein the ratio is determined by quantitative specific amplification of an SK-type and a K-type dehydrin mRNA or cDNA from mRNA or cDNA samples obtained from a plant.

9. The method according to any of claims 1 to 7 wherein the ratio is determined by comparative hybridization of mRNA or cDNA populations obtained from a plant, to more than one dehydrin specific sequences attached to a carrier.

10. The method according to any of the preceding claims wherein the ratio is determined by comparative analysis of protein samples obtained from a plant, to more than one dehydrin type specific antibody, optionally attached to a carrier.

11. A nucleic acid carrier comprising at least two probes capable of selectively hybridizing with SK-type and K-type dehydrins.

**12.** The carrier according to claim 11 wherein the SK-type hybridizing probe is a fragment derived of SEQ ID NO: 1 and/or the K-type hybridizing probe is a fragment derived of SEQ ID NO: 2.

**13.** A kit of parts, comprising at least two or more primers for use in selective amplification of mRNA sequences encoding SK dehydrin Psdhn2 and K dehydrins Psdhn5, and optionally means for visualization of the amplification reaction

**14.** A kit of parts, comprising at least a carrier comprising at least two or more probes capable of selectively hybridizing with SK- type and K-type dehydrins, wherein the SK hybridizing (oligo-) nucleotide probe is a fragment derived of SEQ ID NO: 1 and the K type hybridizing (oligo-) nucleotide probe is a fragment derived of SEQ ID NO: 2, and optionally means for visualization of the hybridization reaction.

**Patentansprüche**

**1.** Verfahren zum Bestimmen der Stresstoleranz in einer Pflanze, umfassend den Schritt des Bestimmens des Verhältnisses zwischen einem SK-Typ-Dehydrin und einem K-Typ-Dehydrin, wobei die zu testende Pflanze eine Gymnosperme ist.

**2.** Verfahren nach Anspruch 1, wobei das Verhältnis mehrmals unter unterschiedlichen Feldbedingungen bestimmt wird.

**3.** Verfahren nach Anspruch 1, wobei das SK-Typ-Dehydrin ein $SK_4$-Dehydrin oder vorzugsweise ein $SK_2$-Dehydrin ist und das K-Typ-Dehydrin ein $K_2$-Dehydrin ist.

**4.** Verfahren nach einem der vorangegangenen Ansprüche, wobei das $SK_2$-Typ-Dehydrin zumindest 85% Identität mit Psdhn2 (SEQ ID NO: 1) hat.

**5.** Verfahren nach einem der vorangegangenen Ansprüche, wobei das $K_2$-Typ-Dehydrin zumindest 85% Identität mit Psdhn5 (SEQ ID NO: 2) hat.

**6.** Verfahren nach einem der vorangegangenen Ansprüche, wobei die zu testende Pflanze aus der Familie Pinaceae ist.

**7.** Verfahren nach einem der vorangegangen Ansprüche, wobei die zu testende Pflanze ausgewählt ist aus dem Genus Pinus, dem Genus Picea, dem Genus Pseudotsuga, dem Genus Tsuga, dem Genus Larix, dem Genus Abies und dem Genus Cedrus.

**8.** Verfahren nach einem der vorangegangenen Ansprüche, wobei das Verhältnis durch quantitative spezifische Amplifikation einer SK-Typ- und einer K-Typ-Dehydrin-mRNA oder -cDNA aus mRNA- oder cDNA-Proben bestimmt wird, die von einer Pflanze erhalten wurden.

**9.** Verfahren nach einem der Ansprüche 1 bis 7, wobei das Verhältnis durch vergleichende Hybridisierung von mRNA- oder cDNA-Populationen, die von einer Pflanze erhalten wurden, an mehr als eine Dehydrin-spezifische Sequenz bestimmt wird, die an einen Träger gebunden sind.

**10.** Verfahren nach einem der vorangegangenen Ansprüche, wobei das Verhältnis durch vergleichende Analyse von aus einer Pflanze gewonnenen Proteinproben mit mehr als einem Dehydrin-Typ-spezifischen Antikörper bestimmt wird, gegebenenfalls gebunden an einen Träger.

**11.** Nukleinsäureträger, umfassend mindestens zwei Sonden, die in der Lage sind, selektiv mit SK-Typ- und K-Typ-Dehydrinen zu hybridisieren.

**12.** Träger nach Anspruch 11, wobei die mit einem SK-Typ hybridisierende Sonde ein Fragment ist, das von SEQ ID NO: 1 abgeleitet ist, und/oder die mit dem K-Typ hybridisierende Sonde ein Fragment ist, das von SEQ ID NO: 2 abgeleitet ist.

**13.** Aus Teilen bestehender Kit, umfassend mindestens zwei oder mehrere Primer für die Verwendung in der selektiven Amplifikation von mRNA-Sequenzen, die SK-Dehydrin Psdhn2 und K-Dehydrin Psdhn5 kodieren; und gegebenenfalls Mittel für die Visualisierung der Amplifikationsreaktion.

**14.** Aus Teilen bestehender Kit, umfassend mindestens einen Träger, der mindestens zwei oder mehr Sonden umfasst, die in der Lage sind, selektiv mit Dehydrin vom SK-Typ und K-Typ zu hybridisieren, wobei die mit SKhybridisierende (Oligo-)Nukleotidsonde ein Fragment ist, das von SEQ ID NO: 1 abgeleitet ist, und die K-Typ-hybridisierende (Oligo-) Nukleotidsonde ein Fragment ist, das von SEQ ID NO: 2 abgeleitet ist; und gegebenenfalls Mittel für die Visualisierung der Hybridisierungsreaktion.

**Revendications**

**1.** Procédé pour déterminer la tolérance au stress d'une plante comprenant l'étape consistant à déterminer le rapport entre une déhydrine de type SK et une déhydrine de type K, la plante devant être testée étant un gymnosperme.

**2.** Procédé conforme à la revendication 1,
selon lequel
le rapport est déterminé à plusieurs reprises dans différentes conditions de champ.

**3.** Procédé conforme à la revendication 1,
selon lequel
la déhydrine de type SK est une déhydrine $SK_4$ ou de préférence une déhydrine $SK_2$ et la déhydrine de type K est une déhydrine $K_2$.

**4.** Procédé conforme à l'une quelconque des revendications précédentes,
selon lequel
la déhydrine $SK_2$ présente au moins 85 % d'identité avec Psdhn2 (SEQ ID NO: 1).

**5.** Procédé conforme à l'une quelconque des revendications précédentes,
selon lequel
la déhydrine $K_2$ présente au moins 85 % d'identité avec Psdhn5 (SEQ ID NO: 2).

**6.** Procédé conforme à l'une quelconque des revendications précédentes,
selon lequel
la plante devant être testée est une plante de la famille des pinacées.

**7.** Procédé conforme à l'une quelconque des revendications précédentes,
selon lequel
la plante devant être testée est choisie parmi le genre Pinus, le genre Picea, le genre Pseudotsuga, le genre Tsuga, le genre Larix, le genre Abies et le genre Cedrus.

**8.** Procédé conforme à l'une quelconque des revendications précédentes,
selon lequel
le rapport est déterminé par amplification quantitative spécifique d'un ARNm ou d'un ADNc d'une déhydrine de type SK et d'une déhydrine de type K à partir d'échantillons d'ARNm ou d'ADNc provenant d'une plante.

**9.** Procédé conforme à l'une quelconque des revendications 1 à 7, selon lequel
le rapport est déterminé par hybridation comparative de populations d'ARNm ou d'ADNc obtenues à partir d'une plante avec plus d'une séquence spécifique de déhydrine liée à un support.

**10.** Procédé conforme à l'une quelconque des revendications précédentes,
selon lequel
le rapport est déterminé par analyse comparative d'échantillons de protéine obtenus à partir d'une plante avec plus d'un anticorps spécifique de type déhydrine le cas échéant lié à un support.

**11.** Support d'acide nucléique comprenant au moins deux échantillons susceptibles de s'hybrider sélectivement avec des déhydrines de type SK et de type K.

**12.** Support conforme à la revendication 11,
dans lequel
l'échantillon d'hybridation de type SK est un fragment dérivé de SEQ ID NO: 1 et/ou l'échantillon d'hybridation de type K est un fragment dérivé de SEQ ID NO: 2.

**13.** Kit de parties comprenant au moins deux ou un plus grand nombre d'amorces destinées à être utilisées pour l'amplification sélective de séquences d'ARNm codant pour la déhydrine SK Psdhn2 et la déhydrine K Psdhn5, et le cas échéant des moyens permettant de visualiser la réaction d'amplification.

**14.** Kit de parties comprenant au moins un support comportant au moins deux ou un plus grand nombre d'échantillons susceptibles de s'hybrider sélectivement avec des déhydrines de type SK et de type K, l'échantillon (oligo) nucléotidique d'hybridation SK étant un fragment dérivé de SEQ ID NO: 1 et l'échantillon (oligo) nucléotidique d'hybridation de type K étant un fragment dérivé de SEQ ID NO: 2, et le cas échéant des moyens permettant de visualiser la réaction d'hybridation.

# Fig 1a

```
Psdhn1                                                      MRRLRMRLASYYP_ACSSAWSRTRPMVTRGNFTPEEAEEKKHTGLIGKLHRTHSSSSSSSSD
Psdhn2  MAEEAPEHKDRGMFGLFGKKKEDEGEQSDHVVQTPA________RTEAASYYPPAPQHGVEQGHGHGHEGQPAREEAEQQKHTG___KLHRSNSSSSSSSSD
Pgdhn1  MAEQAPEHQDRGMFGLFGKKKEEEGRQDDQISQTPATHTHNEAQTEAASYYPTSP_HEVKHGPGHGHEGQLTPEEAEQQKHKGLVGKLHRTHSSSSSSSSD

Psdhn1  EEEEGEKKKEKERKKKEAKDKTNKKLPGDGQHSSEQSDIEEEKKVSLVDKFKEKLPAQPNKGEGEEKVEVEKKAGVKEKLPVQPNKGEREEKAEVEKKV
Psdhn2  EEEEGRKKKEGGRKKKGSKDKTKEKLPGDGKQ______________________Y_PGE________________HEK_____________________
Pgdhn1  EEEEGGKKKEVGRKKKGAKDKTKEKLPSGGHHSSDHCGGEEKKKAGMVDKIKEKLPGH_______________QEKLPAGGEHSSDQCGTKEEKKT

Psdhn1  GLLDKIKE_LPGGHNQKGRGRE_KKKKKQKWRKRRVLIDKIKEKLPGHHTKKEGEEEEEKKQNY
Psdhn2  ______________________CYSRQHGGEEEKKAGMLDKIKAKLPGQHNKNDGEQ
Pgdhn1  GLLDKIKDKLPGHQEKLPGGGQHSDQYGGKQESKMGLLDKIKEKLPGHENKNDGEEEEEKKHPH
```

# Fig 1b

```
Psdhn3  MAQPAHQDRGMFG___KKKEDEGKQCDQ____TEGGRCEEGKKPGMVDKIKEKIPGQQQKLPGDGKQCCGQPGGEEEKKPGMVDKIKQKLPGQQNKTGCD
Psdhn4  MAQPAHQDRGMFG___KKKEDEGRQCDQ____TEGGRCEEGKKPGMVDKIKEKIPGQQQKLPGDGKQCGGQPGREEEKKPGMVDKIKQKLPGQQNKNDVE
Psdhn5  MAE_EQQDRGMFGLLGKKKEDEGKQCDQ____TEGGRCEEGKKPGMVDKIKEKILGQQQKLPGDGKQCGGQPGREEEKKPGMVDKIKQKLPGQQNKNDVE
Psdhn6  MAE_EQHDRGMFGLLCKKKEDEGKQCDH____TEGGRCEEGKKPGMVDKIKEKILGQQPKLPRDRKQCGCHPGRVEEKRPRMVDKIKQKLPGHQNKNDVE
Psdhn7  MAE_EQQDRGMFGLLGKKKEDEGKQCDQ____TEGGRCEEGKKPGMVDKVKEKIPGQQQKLPGDGKQCCGQPGGEEEKKPGMVDKIKQKLPGQQNKTGCD
Psdhn8  MAE_EQQDRGMFGDLGKKKEDEGKQCDQGPPRTEVGRCEEGKKPGMVDKIKEKIPGQQQKLPGDGKQCGGQPGREEEKKPGMVDKIKQKLPGQQN
```

# Fig 1c

| | | | |
|---|---|---|---|
| Consensus | *P. sylvestris* | *Psdhn1*, *Psdhn2* | DK*K*KLPG |
| | *P. taeda* | Acc.no. BG039896,AW981612,H75267 | |
| | *P. pinaster* | Acc.no. BX253143,BX253049,BX250897 | |
| Consensus | *P. sylvestris* | *Psdhn3-8* | EKKPGMVDKIK*KLPG |
| | *P. taeda* | Acc.no. BG040304 | |

Fig 2a

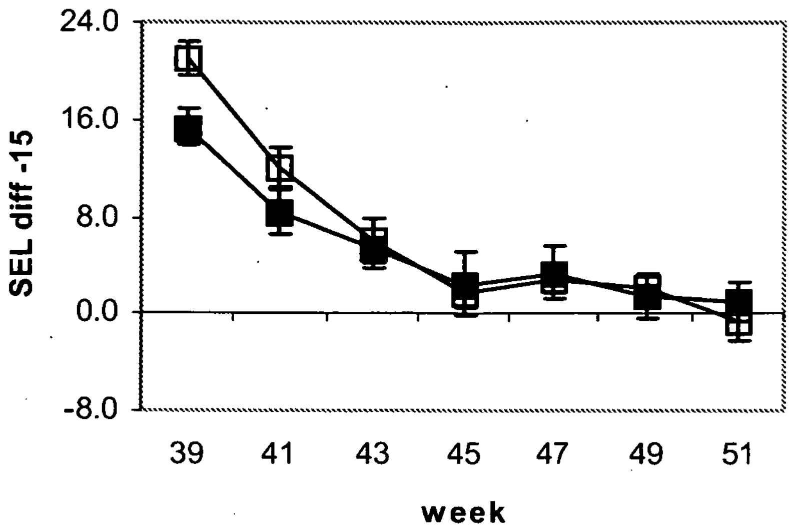
24.0
16.0
8.0
0.0
-8.0
SEL diff -15
39
41
43
45
47
49
51
week

Fig 2b

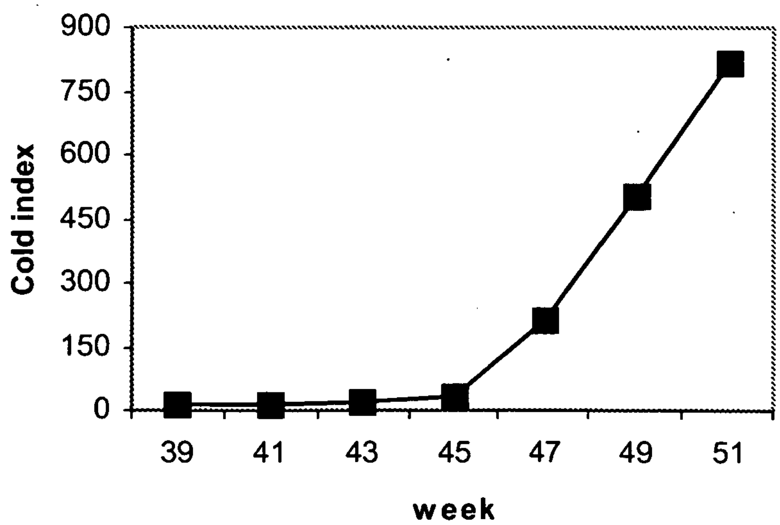
900
750
600
450
300
150
0
Cold index
39
41
43
45
47
49
51
week

Fig 3

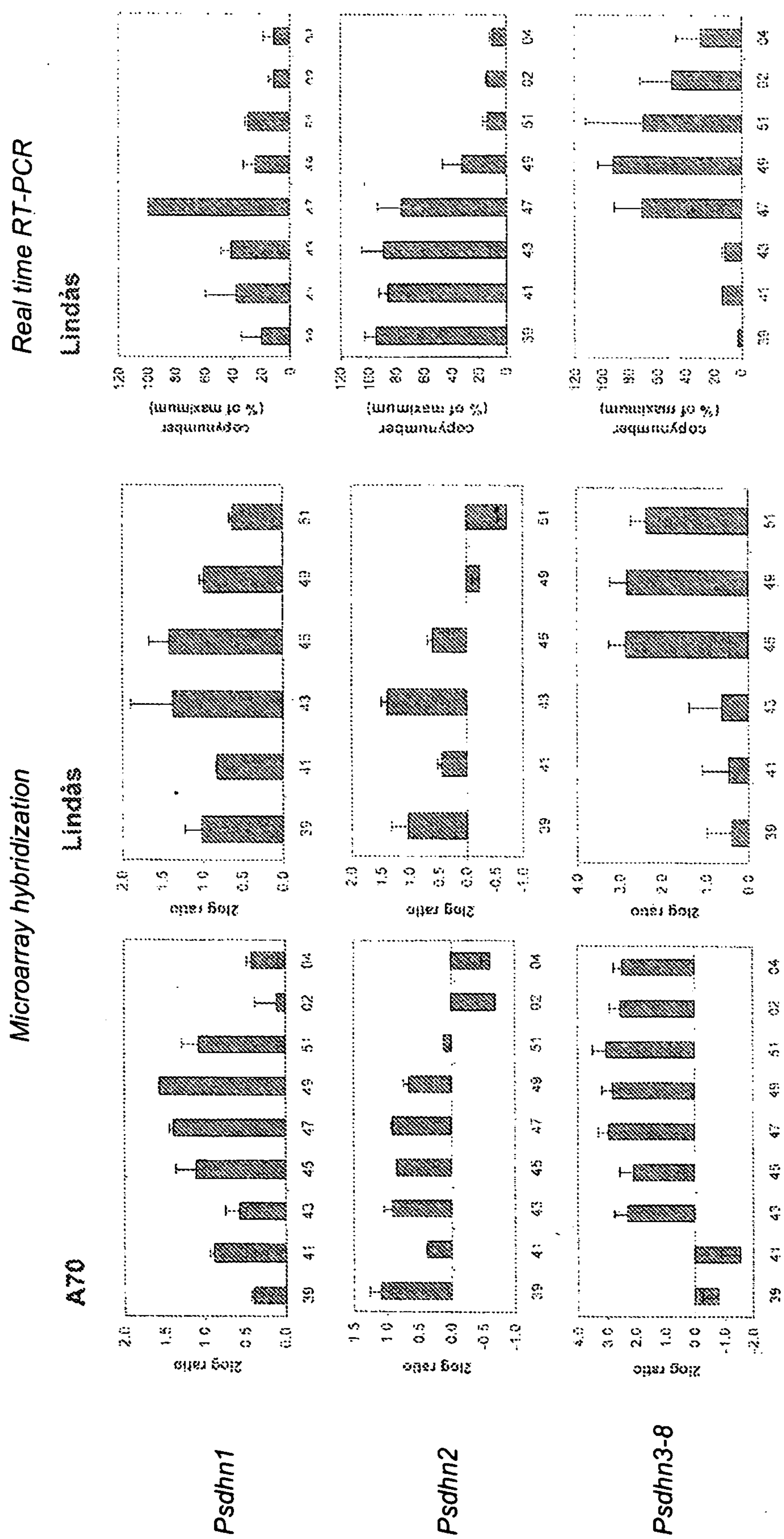
Microarray hybridization
Real time RT-PCR
A70
Lindås
Lindås
Psdhn1
Psdhn2
Psdhn3-8
2log ratio
copynumber (% of maximum)

Fig 4a

| | | Ct-values | | | |
|---|---|---|---|---|---|
| **A70** | **stage** | ***Psdhn1*** | ***Psdhn2*** | ***Psdhn5*** | **18S** |
| week 41 | I | 21.6 | 16.8 | 19.1 | 10.9 |
| week 43 | IIA | 20.5 | 15.5 | 14.4 | 10.9 |
| week 49 | IIA | 19.7 | 15.7 | 12.6 | 10.8 |
| week 02 | IIB | 20.8 | 18.7 | 13.5 | 10.7 |
| **Lindås** | | | | | |
| week 43 | I | 20.3 | 15.9 | 17.0 | 11.0 |
| week 47 | IIA | 20.0 | 16.7 | 13.9 | 10.9 |
| week 51 | IIB | 21.1 | 18.7 | 14.4 | 11.0 |

Fig 4b

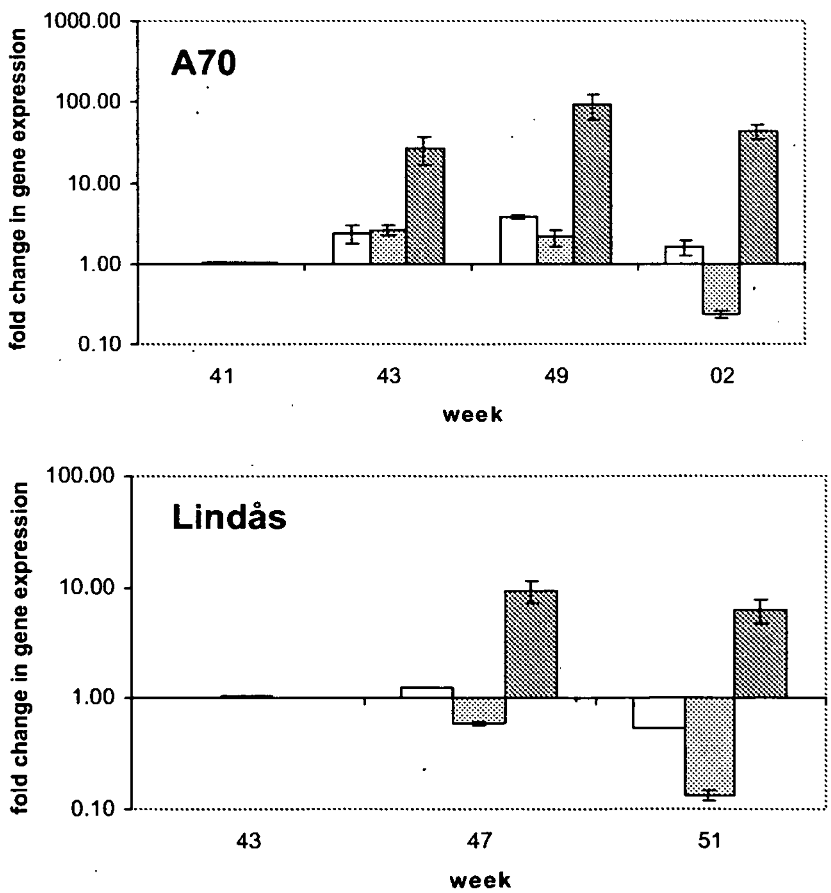

*Fig 4c*

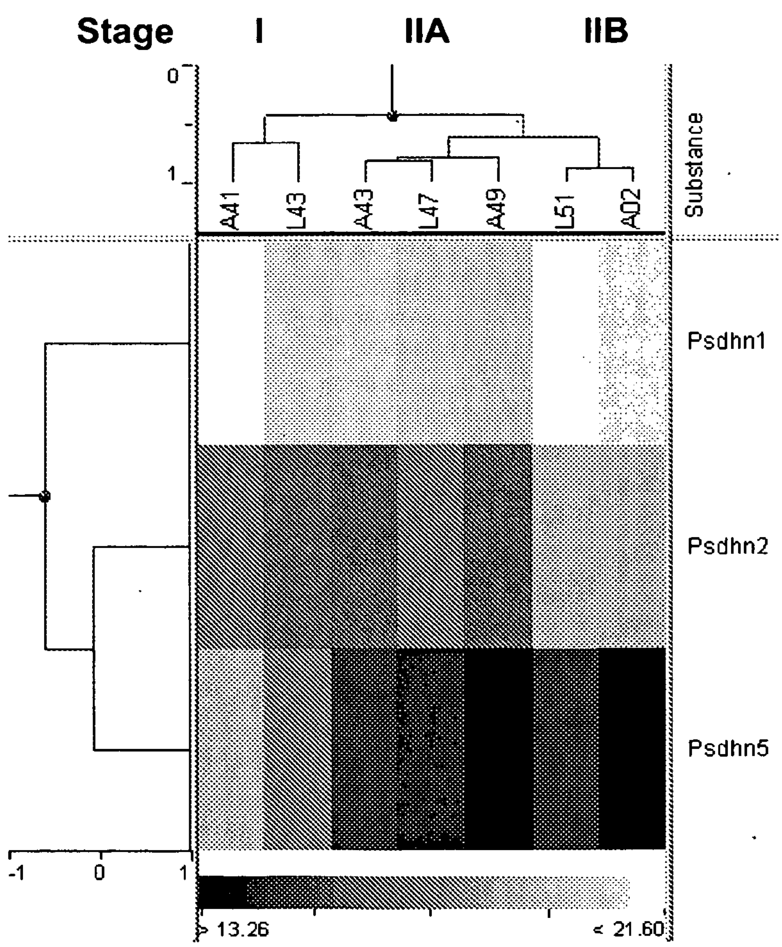
Stage
I
IIA
IIB
0
1
A41
L43
A43
L47
A49
L51
A02
Substance
Psdhn1
Psdhn2
Psdhn5
-1
0
1
> 13.26
< 21.60

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6501006 B


### Non-patent literature cited in the description


- **Labhilili et al.** *Plant Science,* 1995, vol. 112, 219-30
- **Lopez et al.** *Crop Science,* 2003, vol. 43, 577-82
- **A.H. Fitter ; R.K.M. Hay.** Environmental Physiology of Plants. Academic Press, 1987
- A-Z of Quantitative PCR, IUL Biotechnology series. 2005
- **R.B. Stoughton.** Applications of DNA Microarrays in Biology. *Annu.Rev.Biochem.,* 2005, vol. 74, 53-82
- **David Bowtell ; Joseph Sambrook.** DNA Microarrays: A Molecular Cloning Manual. Cold Spring Harbor laboratory press, 2003
- **Ausubel et al.** Current protocols in Molecular Biology. Wiley Interscience, 2004
- **Close,T.J. ; Fenton, R.D. ; Moonan, F.** *Plant Mol.Biol.,* 1993, vol. 23, 279-286
- Current Protocols in Immunology. Wiley Interscience, 2004
- **Tian et al.** *Planta,* 2001, vol. 213 (6
- **Tang ; Tian.** *J. Exp. Botany,* 2003, vol. 54 (383
- **Aronen TS et al.** *Transgenic Res.,* 2003, vol. 12 (3
- **Grant JE et al.** *Plant Cell reports,* 2004, vol. 22 (12
- Computational Molecular Biology. Oxford University Press, 1988
- Biocomputing: Informatics and Genome Projects. Academic Press, 1993
- Computer Analysis of Sequence Data, Part I. Humana Press, 1994
- **von Heine, G.** Sequence Analysis in Molecular Biology. Academic Press, 1987
- Sequence Analysis Primer. M Stockton Press, 1991
- **Carillo, H. ; Lipman, D.** *SIAM J. Applied Math.,* 1988, vol. 48, 1073
- **Devereux, J. et al.** *Nucleic Acids Research,* 1984, vol. 12 (1), 387
- **Altschul, S. F. et al.** *J. Mol. Biol.,* 1990, vol. 215, 403-410
- **Altschul, S. et al.** *J. Mol. Biol.,* 1990, vol. 215, 403-410
- **Needleman ; Wunsch.** *J. Mol. Biol.,* 1970, vol. 48, 443-453
- **Hentikoff ; Hentikoff.** *Proc. Natl. Acad. Sci. USA.,* 1992, vol. 89, 10915-10919
- **Aharoni, A. ; L.C. Keizer ; H.J. Bouwmeester ; Z. Sun ; M. Alvarez Huerta ; H.A. Verhoeven ; J. Blaas ; A.M. van Houwelingen ; R.C. de Vos ; H. van der Voet.** Identification of the SAAT gene involved in strawberry flavor biogenesis by use of DNA microarrays. *Plant Cell,* 2000, vol. 12 (5), 647-662
- Simple and rapid bacterial protein and DNA diagnostic methods based on signal generation with colloidal carbon particles. **Aart van Amerongen ; Marjo Koets.** Rapid methods for biological and chemical contaminants in food and feed. Wageningen Academic Publishers, 2005, 105-126
- **Alsheikh, M.K. ; B.J. Heyen ; S.K. Randall.** Ion binding properties of the dehydrin ERD14 are dependent upon phosphorylation. *J.Biol.Chem.,* 2003, vol. 278 (42), 40882-40889
- **Artlip, T.S. ; A.M. Callahan ; C.L. Basset ; M.E. Wisniewski.** Seasonal expression of a dehydrin gene in sibling deciduous and evergreen genotypes of peach (Prunus persica (L.) Batsch). *Plant Mol. Biol.,* 1997, vol. 33, 61-70
- Conifer bud dormancy and stress resistance: A forestry perspective. **Bigras, F.J.** Plant dormancy. Physiology, Biochemistry and Molecular Biology. 1996, 171-192
- **Campbell, S.A. ; T.J. Close.** Dehydrins: genes, proteins, and associations with phenotypic traits. *New Phytol.,* 1997, vol. 137, 61-74
- **Chang, S. ; J. Puryear ; J. Cairney.** A simple and efficient method for isolating RNA from pine trees. *Plant Mol. Biol. Rep.,* 1993, vol. 11 (2), 113-116

- **Choi, D.W. ; B. Zhu ; T.J. Close.** The barley (Hordeum vulgare L.) dehydrin multigene family: sequences, allele types, chromosome assignments, and expression characteristics of 11 Dhn genes of cv Dicktoo. *Theor. Appl. Genet.,* 1999, vol. 98, 1234-1247
- **Christersson, L.** The influence of photoperiod and temperature on the development of frost hardiness in seedlings of Pinus sylvestris and Picea abies. *Physiol.Plant.,* 1978, vol. 44, 288-294
- **Close, T.J. ; R.D. Fenton ; F. Moonan.** A view of plant dehydrins using antibodies specific to the carboxy terminal peptide. *Plant Mol. Biol.,* 1993, vol. 23, 279-286
- **Close, T.J.** Dehydrins: Emergence of a biochemical role of a family of plant dehydration proteins. *Phys. Plant.,* 1996, vol. 97, 795-803
- **Close, T.J.** Dehydrins: A commonalty in the response of plant to dehydration and low temperature. *Phys. Plant.,* 1997, vol. 100, 291-296
- **Doom, W.G. ; P.A. Balk ; A.M. van Houwelingen ; F.A. Hoeberichts ; R.D. Hall ; O. Vorst ; C. van der Schoot ; M.F. van Wordragen.** Gene expression during anthesis and senescence in Iris flowers. *Plant.Mol.Biol.,* 2003, vol. 53, 845-863
- Bud dormancy, frost hardiness, and frost drought in seedlings of Pinus sylvestris and Picea abies. **Dormling I.** Advances in plant cold hardiness. CRC Press, 1993, 285-298
- **Ekberg I. ; G. Eriksson ; d I. Dormling.** Photoperiodic reactions in conifer species. *Holarctic Ecology,* 1979, vol. 2, 255-263
- **Heyen. B.J. ; M.K. Alsheikh ; E.A. Smith ; C.F. Torvik ; D.F. Seals ; S.K. Randall.** The Calcium-binding activity of a vacuole-associated, dehydrin-like protein is regulated by phosphorylation. *Plant Physiol.,* 2002, vol. 130, 675-687
- **Jarvis, S.B. ; M.A. Taylor ; M.R. Macleod ; H.V. Davies.** Cloning and characterisation of the cDNA clones of three genes that are differentially expressed during dormancy-breakage in the seeds of Douglas fir (Pseudotsuga menzeisii). *J. Plant Physiol.,* 1996, vol. 147, 559-566
- **Karlson D.T. ; T. Fujino ; S. Kimura ; K. Baba ; T. Itoh ; E.N. Ashworth.** Novel plasmodesmata association of dehydrin-like proteins in cold acclimated red-osier dogwood (Cornus sericea). *Tree Physiol.,* 2003, vol. 23, 759-767
- **Koag, M. ; R.D. Fenton ; S. Wilkens ; T.J. Close.** The binding of Maize DHN1 to lipid vesicles. Gain of structure and lipid specificity. *Plant Physiol.,* 2003, vol. 131, 309-316
- **Marjo Koets ; Nathalie Barbier ; Emil Wolbert ; Hans Mooibroek ; Aart van Amerongen.** Rapid and simple one-step and mini-array methods to detect and quantify amplified DNA and proteins using ligand-labeled colloidal particles. *Proceedings EURO FOOD CHEM XII - Strategies for Safe Food,* 24 September 2003, 121-124
- **Kontunen-Soppela, S. ; K. Laine.** Seasonal fluctuation of dehydrins is related to osmotic status in Scots pine needles. *Trees,* 2001, vol. 15, 425-430
- **Diane Kozwich ; Kristine A. Johansen ; Keli Landau ; Christopher A. Roehl ; Sam Woronoff ; Patrick A. Roehl.** Development of a novel, rapid integrated Cryptosporidium parvum detection assay. *Applied and Environmental Microbiology,* 2000, vol. 66, 2711-2717
- **Lang, G.A.** Dormancy: a new universal terminology. *HortScience,* 1987, vol. 22, 817-820
- **Levi, A. ; G.R. Panta ; C.M. Parmentier ; M.M. Muthalif ; R. Arora ; S. Shanker ; L.J. Rowland.** Complementary DNA cloning, sequencing and expression of an unusual dehydrin from blueberry floral buds. *Physiol.Plant.,* 1999, vol. 107, 98-109
- **Lim, C.C. ; S.L. Krebs ; R. Arora.** A 25-kDa dehydrin associated with genotype- and age-dependent leaf freezing-tolerance in Rhododendron: a genetic marker for cold hardiness?. *Theor. Appl. Genet.,* 1999, vol. 99, 912-920
- **Lindström, A. ; L. Häkansson.** *EC-metoden - et sätt at bestämma skogplantors lagringsbarhet. Sveriges Lantbruksuniversitet, Institutionen for skogsproduktion. Stencil nr. 95,* 1996, 30
- **Livak K.J. ; T.D. Schmittgen.** Analysis of relative gene expression data using real-time quantitative PCR and the 2-ΔΔCT method. *Methods,* 2001, vol. 25, 402-408
- **Momma, M. ; S. Kaneko ; K. Haraguchi ; U. Matsukura.** Peptide mapping and assessment of cryoprotective activity of 26/27-kDa dehydrin from soybean seeds. *Biosci.Biotechnol.Biochem.,* 2003, vol. 67 (8), 1832-1835
- **McKay, H.M.** Electrolyte leakage from fine roots of conifer seedlings: a rapid index of plant vitality following cold storage. *Can.J.For.Res.,* 1992, vol. 22, 1371-1377
- **Nylander, M. ; J. Svensson ; E.T. Palva ; B.V. Welin.** Stress-induced accumulation and tissue-specific localization of dehydrins in Arabidopsis thaliana. *Plant Mol. Biol.,* 2001, vol. 45, 263-279
- **Perks, M.P. ; H.M. Mckay.** Morphological and physiological differences in Scots pine seedlings of six different origins. *Forestry,* 1997, vol. 70 (3), 223-232

- **Porat, R. ; D. Pavoncello ; S. Lurie ; T.G. Mccollum.** Identification of a grapefruit cDNA belonging to a unique class of citrus dehydrins and characterization of its expression patterns under temperature stress conditions. *Physiol.Plant.,* 2002, vol. 115, 598-603
- **Repo, T. ; G. Zhang ; A. Ryyppö ; R. Rikala ; M. Vuorinen.** The relation between growth cessation and frost hardening in Scots pins of different origins. *Trees,* 2000, vol. 14, 456-464
- **Richard, S. ; M. Morency ; C. Drevet ; L. Jouanin ; A. Seguin.** Isolation and characterization of a dehydrin gene from white spruce induced upon wounding, drought and cold stress. *Plant Mol. Biol.,* 2000, vol. 43, 1-10
- **Rinne, P.L.H. ; P.L.M. Kaikuranta ; L.H.W. van der Plas ; C. van der Schoot.** Dehydrins in cold-acclimated apices of birch (Betula pubescens Ehrh.): production, localization and potential role in rescuing enzyme function during dehydration. *Planta,* 1999, vol. 209, 377-388
- **Rowland L.J. ; R. Arora.** Proteins related to endodormancy (rest) in woody perennials. *Plant Science,* 1997, vol. 126, 119-144
- **Stattin, E. ; Hellqvist, C. ; A. Lindström.** Storability and root freezing tolerance of Norway spruce (Picea abies) seedlings. *Can.J.For.Res.,* 2000, vol. 30, 964-970
- **Wang, W. ; D. Pelah ; T. Alergand ; O. Shoseyov ; A. Altman.** Characterization of SP1, a stress-responsive, boiling soluble, homo-oligomeric protein from aspen. *Plant Physiology,* 2002, vol. 130, 865-875
- **Welling, A. ; T. Moritz ; E.T. Palva ; O. Junttila.** Independent activation of cold acclimation by low temperature and short photoperiod in hybrid aspen. *Plant Physiology,* 2002, vol. 129, 1633-1641
- **Welling, A. ; P. Rinne ; A. Viherä-Aarnio ; S. Kontunen-Soppela ; P. Heino ; E.T. Palva.** Photoperiod and temperature differentially regulate the expression of two dehydrin genes during overwintering of birch (Betula pubescens Ehrh.). *J.Exp.Botany,* 2004, vol. 55 (396), 507-516
- **Wise, M.J. ; A. Tunnacliffe.** POPP the question: what do LEA proteins do?. *Trends in Plant Science,* 2004, vol. 9 (1), 13-17
- **Zhu, B. ; D.W. Choi ; R. Fenton ; T.J. Close.** Expression of the barley dehydrin multigene family and the development of freezing tolerance. *Mol.Gen.Genet.,* 2000, vol. 264, 145-153

SEQUENCE LISTING

<110> Agrotechnology & Food Innovations, Wageningen

<120> Methods and means for determining stress tolerance in plants

<130> P218055 EP

<160> 18

<170> PatentIn version 3.3

<210> 1

<211> 918
<212> DNA
<213> Pinus sylvestris

<400> 1

```
ctgaaggagt agaaaatcta ctgcgttctc tgcatttgtt gtttattata tcgtccgcaa    60
tttttacgaa gttatttact cgtcttagtg ttttaaatta gtattaagat ggcggaagaa   120
gcaccagagc acaaggaccg cggtatgttc ggcttgttcg gcaaaaagaa ggaagatgag   180
ggaaagcaaa gtgatcacgt tgtgcagact cctgctcgga ctgaggctgc ctcttattat   240
ccacctgctc ctcagcatgg agtcgaacaa ggccacggtc atggtcacga ggggcagttt   300
gcccgtgagg aagctgagca acagaaacac acgggaaagc ttcaccgctc gaacagttcc   360
agctccagct cttcgagcga tgaagaggag gaaggcagaa agaagaaaga aggcggaagg   420
aagaagaaag ggtcaaagga caagaccaag gaaaagctcc ccggggacgg aaagcagtat   480
cccggtgagc atgaaaagca gtattcccgt cagcatgggg gtgaagagga aaagaaggcg   540
ggtatgcttg ataaaatcaa agcgaagctg ccgggacaac ataacaagaa tgatggggaa   600
cagtaagaga agaagcaccc cctgcccata taatatgtga atctgtgtct actcgttcag   660
ggcgttgctt aggagcatgc atggatgaga ataatggtac gtgcgtgttg tggggttttg   720
attagtaatg tgttgtgtta caagagggga taatgtaatc cgatcaatcc gcattgccat   780
tggtgatgtc ggaatttgtg gttttataat aaatggggtt tcggtacgag cttaccttca   840
gcgtctgtcc gatgtatcga ttaaagttat gaatatatta taatcagtat tttcatgtga   900
aaaaaaaaaa aaaaaaaa                                                 918
```

<210> 2
<211> 647
<212> DNA
<213> Pinus sylvestris

<400> 2

```
aatttgttgt ttgttatatc gtctgcaatt tttaccaagt tttttactcg ttattaagat      60
ggcggaagaa caacaggacc gcggtatgtt cggcttattg ggcaaaaaga aggaagatga     120
gggaaagcaa tgtgatcaga ctgagggtgg tagatgtgaa gaggggaaga agccgggaat     180
ggtagataaa atcaaagaga agattccggg acaacagcaa aagctccccg gagacggaaa     240
gcagtgtggc ggtcagcctg ggcgtgaaga ggaaaagaag ccaggtatgg ttgataaaat     300
caaacagaag ctgccgggac aacaaaataa gaatgacgtg gagtaagaga agatagtgta     360
aagaagaccc cctgcccatc taatatgtga atctgtgtac actctcgttc agggcattgc     420
ttaggagcat gcatggatga gaataatgtt atgtgcgtgt tgattagtaa tgtgttgtgt     480
tacaagaggg ggatcaaagt tacgactgta atccgatggt gaggtcggta tttgcggttt     540
tataataaat agggtttcga gtaccatctt ttacctgagc gtgtttccga tttatcggtt     600
gaagttatga atatagttta tgtgaccaaa ctttcagcaa tgttcaa                   647
```

<210> 3
<211> 681
<212> DNA
<213> Pinus sylvestris

<220>
<221> misc_feature
<222> (112)..(112)
<223> n is a, c, g, or t

<400> 3

```
tgttttctct aacatttgtg attcaagcct gtgcgtgggg tattcgaatc cagtcgccgc      60
atatatagtc ggtgtgtctt tttgtgtgta taagcatgtc tgagggacag cngtcaccac     120
ctcttcaacc gcaaaaagga ggaggacaac gttaattctg gctctgctga ccagggctcc     180
tatgggtcct ctgattacca ggctggctct ggtaataacg ctggctctgg ttataacgcc     240
gacactggtt ataaggccga ttacggcaat gatggtcagc aaggtcaata tgatggtcag     300
caaggtcaat cacagaacta tgggggagcc caaaaggagg agaactatgg gggagcccaa     360
aaggaagtga aacaagacca gcgcaaggag cgcttcgggg agctcggaac cgcggctgct     420
ggaggctatg cactgtatga gaggaatgag gcaaaaaagg atcctgagaa tgctaggagg     480
cacaagacag aggaggaggt cgctgctgcg ggcgctgtgg gcaccggtgg gtacgcgttc     540
cacgagcgcc acgagaagaa acgagacgag gaaaccgagg aagccgaggg tggccgcaag     600
cctcgccaca acctcttctg aatttggcca tgcctctcta atggtggact tttggagcag     660

aagccaatta tagcagttgg c                                               681
```

<210> 4
<211> 590
<212> DNA
<213> Pinus sylvestris

<220>
<221> misc_feature
<222> (115)..(115)
<223> n is a, c, g, or t

<220>
<221> misc_feature
<222> (123)..(123)
<223> n is a, c, g, or t

<220>
<221> misc_feature
<222> (134)..(134)
<223> n is a, c, g, or t

<220>
<221> misc_feature
<222> (136)..(136)
<223> n is a, c, g, or t

<220>
<221> misc_feature
<222> (142) .. (142)
<223> n is a, c, g, or t

<220>
<221> misc_feature
<222> (147) .. (147)
<223> n is a, c, g, or t

<220>
<221> misc_feature
<222> (150)..(150)
<223> n is a, c, g, or t

<220>
<221> misc_feature
<222> (152) .. (152)
<223> n is a, c, g, or t

<220>
<221> misc_feature
<222> (155)..(155)
<223> n is a, c, g, or t

<220>
<221> misc_feature
<222> (158)..(158)
<223> n is a, c, g, or t

<220>
<221> misc_feature
<222> (160) .. (160)

<223> n is a, c, g, or t

<220>
<221> misc_feature
<222> (163) .. (163)
<223> n is a, c, g, or t

<220>
<221> misc_feature
<222> (169)..(169)
<223> n is a, c, g, or t

<220>
<221> misc_feature
<222> (172)..(172)
<223> n is a, c, g, or t

<220>
<221> misc_feature
<222> (185)..(185)
<223> n is a, c, g, or t

<220>
<221> misc_feature
<222> (193)..(193)
<223> n is a, c, g, or t

<220>
<221> misc_feature
<222> (195) .. (195)
<223> n is a, c, g, or t

<220>
<221> misc_feature
<222> (209) .. (209)
<223> n is a, c, g, or t

<220>
<221> misc_feature
<222> (224) .. (224)
<223> n is a, c, g, or t

<220>
<221> misc_feature
<222> (226)..(226)
<223> n is a, c, g, or t

<220>
<221> misc_feature
<222> (232)..(233)
<223> n is a, c, g, or t

<220>
<221> misc_feature
<222> (236) .. (236)
<223> n is a, c, g, or t

<220>

<221> misc_feature
<222> (238)..(238)
<223> n is a, c, g, or t

<220>
<221> misc_feature
<222> (244) .. (244)
<223> n is a, c, g, or t

<220>
<221> misc_feature
<222> (253) .. (253)
<223> n is a, c, g, or t

<220>
<221> misc_feature
<222> (260)..(260)
<223> n is a, c, g, or t

<220>
<221> misc_feature
<222> (270)..(271)
<223> n is a, c, g, or t

<220>
<221> misc_feature
<222> (277) .. (277)
<223> n is a, c, g, or t

<220>
<221> misc_feature
<222> (289)..(289)
<223> n is a, c, g, or t

<220>
<221> misc_feature
<222> (292).. (292)
<223> n is a, c, g, or t

<220>
<221> misc_feature
<222> (297)..(297)
<223> n is a, c, g, or t

<220>
<221> misc_feature
<222> (304) .. (304)
<223> n is a, c, g, or t

<220>
<221> misc_feature
<222> (306)..(306)
<223> n is a, c, g, or t

<220>
<221> misc_feature
<222> (317)..(317)
<223> n is a, c, g, or t

<220>
<221> misc_feature
<222> (321)..(321)
<223> n is a, c, g, or t

<220>
<221> misc_feature
<222> (327)..(327)
<223> n is a, c, g, or t

<220>
<221> misc_feature
<222> (331) .. (331)
<223> n is a, c, g, or t

<220>
<221> misc_feature
<222> (336)..(336)
<223> n is a, c, g, or t

<220>
<221> misc_feature
<222> (338)..(338)
<223> n is a, c, g, or t

<220>
<221> misc_feature
<222> (343) .. (343)
<223> n is a, c, g, or t

<220>
<221> misc_feature
<222> (348) .. (348)
<223> n is a, c, g, or t

<220>
<221> misc_feature
<222> (353)..(353)
<223> n is a, c, g, or t

<220>
<221> misc_feature
<222> (361)..(361)
<223> n is a, c, g, or t

<220>
<221> misc_feature
<222> (368)..(368)
<223> n is a, c, g, or t

<220>
<221> misc_feature
<222> (389)..(389)
<223> n is a, c, g, or t

<220>
<221> misc_feature
<222> (398)..(398)

<223> n is a, c, g, or t

<220>
<221> misc_feature
<222> (401) .. (401)
<223> n is a, c, g, or t

<220>
<221> misc_feature
<222> (413) .. (413)
<223> n is a, c, g, or t

<220>
<221> misc_feature
<222> (419) .. (419)
<223> n is a, c, g, or t

<220>
<221> misc_feature
<222> (424) .. (424)
<223> n is a, c, g, or t

<220>
<221> misc_feature
<222> (448) .. (448)
<223> n is a, c, g, or t

<220>
<221> misc_feature
<222> (460) .. (460)
<223> n is a, c, g, or t

<220>
<221> misc_feature
<222> (465) .. (465)
<223> n is a, c, g, or t

<220>
<221> misc_feature
<222> (470)..(470)
<223> n is a, c, g, or t

<220>
<221> misc_feature
<222> (496) .. (496)
<223> n is a, c, g, or t

<220>
<221> misc_feature
<222> (533)..(533)
<223> n is a, c, g, or t

<220>
<221> misc_feature
<222> (542)..(542)
<223> n is a, c, g, or t

<220>

<221> misc_feature
<222> (556) .. (556)
<223> n is a, c, g, or t

<220>
<221> misc_feature
<222> (563)..(563)
<223> n is a, c, g, or t

<400> 4

```
cgaatacagc agtaacaggc gtggtggtat tgcaggaggc gtctttgcaa acgccttctt     60
catccgattt cctacgttcg ccgtttcttc gcagtttttc tctttttttt ttttnagggt    120
ttnttttcct tcgncnccag cnaaaangan anagngcntn tcnatccana tnggacaggc    180
cggtntccag gtngnaaatg cttgctggna actttactgc ctcnancacg gnnttnancc    240
tganggccaa atncccagcn acaaaactgn ngggggngga aacaatgcnt tnaacanttt    300
tttnancaaa actgganccg naaagcntgt nccccncnct gtntttgnaa atntggaccc    360
nactgtcntt gacaaggtac ggactggtnc ttaccgtnag nttttccatc ctnaacagnt    420
tatnagcggc aaggaaaatg cggccaanaa ctttgcccgn ggtcnttacn ccattggcaa    480
ggaaattggg gacctntgcc tggaccgtat tcgcaagctg gctgacaact gcncgggtct    540
tnaaggattc ctcgtnttca acncagttgg gggaggaact ggttctggtc                590
```

<210> 5
<211> 537
<212> DNA
<213> Pinus sylvestris

<400> 5

```
aatttgttgt ttgttatatc gtctgcaatt tttactaagt tttttactcg ttattaagat     60
ggcgcaacca gcacaccagg accgcggtat gttcggcaaa aagaaggaag atgagggaaa    120
gcaatgtgat cagactgagg gtggtagatg tgaagagggg aagaaaccgg gaatggtaga    180
taaaatcaaa gagaagattc cgggacaaca gcaaaagctc cccggagacg gaaagcagtg    240
ttgcggtcag cctggaggtg aagaggaaaa gaagccaggt atggttgata aaatcaaaca    300
gaagctgccg ggacaacaaa acaagactgg ttgtgattaa gagaagatag tgtagtctgt    360
gtttactcct ccagagcttg ggtgaggagc atggatgaga ataatcgtat gtgcgtttgt    420
ggtattgtaa taaatgggac ttcgggtaca atcgtacctg agcgtctgtc cgatgtatcg    480
attaaagtta tgaatatatt ataatagttt tttcatatta aaaaaaaaaa aaaaaaa        537
```

<210> 6
<211> 337
<212> DNA

<213> Pinus sylvestris

<400> 6

```
aagatggcgc aaccagcaca ccaggaccgc ggtatgttcg gcaaaaagaa ggaagatgag      60
ggaaggcaat gtgatcagac tgagggtggt agatgtgaag aggggaagaa gccgggaatg     120
gtagataaaa tcaaagagaa gattccggga caacagcaaa agctccccgg agacggaaag     180
cagtgtggcg gtcagcctgg gcgtgaagag gaaaagaagc caggtatggt tgataaaatc     240
aaacagaagc tgccgggaca acaaaataag aatgacgtgg agtaagagaa gatagtgtaa     300
agaagacccc ctgcccatct aatatgtgaa tctgtgt                              337
```

<210> 7
<211> 409
<212> DNA
<213> Pinus sylvestris

<400> 7

```
gggctatttg ctgtttgtta tatcgtctgc agtttttacc aacttttta ctcgttatta      60
agatggcgga agaacaacac gaccgcggta tgttcggctt attgtgcaaa aagaaggaag     120
atgagggaaa gcaatgtgat cacactgagg gtggtagatg tgaagagggg aagaagccgg     180
gaatggtcga taaaatcaaa gagaagattc tgggacaaca gccaaagctt ccccgagacc     240
gaaagcagtg tggctgtcac cctggtcgtg tagaggaaaa gagaccacgt atggttgata     300
aaatcaaaca gaagctgccg ggacatcaaa ataaaaatga cgtggagtaa gagaagatat     360
tgtttagaag accccccctgc cctctcatat gtgaatatgt gtacactct               409
```

<210> 8
<211> 559
<212> DNA
<213> Pinus sylvestris

<400> 8

```
gcgttctgtc aatttgttgt ttgttatatc gtctgcattt tttaccaagt tttttactcg      60
```

```
ttattaagat ggcggaagag caacaggacc gcggtatgtt cggcttattg ggcaaaaaga    120
aggaagatga gggaaagcaa tgtgatcaga ctgagggtgg tagatgtgaa gaggggaaga    180
agccgggaat ggtagataaa gtcaaagaga agattccggg acaacagcaa aagctccccg    240
gagacggaaa gcagtgttgc ggtcagcctg gaggtgaaga ggaaaagaag ccaggtatgg    300
ttgataaaat caaacagaag ctgccgggac aacaaaacaa gactggttgt gattaagaga    360
agatagtgta gtctgtgttt actcctccag agcttgggtg aggagcatgg atgagaataa    420
tagtatgtgc gtttgtgttt ggggtattat aataaatggg acttcgggta caagcgtacc    480
tgagcgtctg tccgatgtat caattaaagt tatgaatata ctataaaagt tttttcatgt    540
taaaaaaaaa aaaaaaaaa                                                 559
```

<210> 9
<211> 341
<212> DNA
<213> Pinus sylvestris

<400> 9

```
atttgttgtt tgttatatcg tctgcatttt tcaccaaagt tatttactcg ttattaagat     60
ggcggaagag caacaggacc gcggtatgtt cggcttatta ggcaaaaaga aggaagatga    120
gggaaagcaa tgtgatcagg gtcctcctcg gactgaggtt ggtagatgtg aagaggggaa    180
gaagccggga atggtagata aaatcaaaga gaagattccg ggcaacagc aaaagctccc     240
cggagacgga aagcagtgtg gcggtcagcc tgggcgtgaa gaggaaaaga agccaggtat    300
ggttgataaa atcaaacaga agctgccggg acaacaaaat a                        341
```

<210> 10
<211> 1143
<212> DNA
<213> Pinus sylvestris

<400> 10

```
catcacccaa agtgaggctc agaatgagga ggctcagaat gaggcttgct tcttattatc     60
cagcttgctc ctccgcatgg agccgaacac ggcccatggt cacgaggggc aactttaccc    120
ctgaggaagc tgaggaaaag aaacacacgg gtttgatagg aaagcttcac cgcacccaca    180
gttccagctc cagctcttct agtgatgaag aggaagaagg cgagaaaaag aaagagaaag    240
agagaaagaa gaaagaggca aaggacaaga ccaataaaaa gctacctgga gatggacagc    300
attcttcaga tcagtctggg attgaagaag agaagaaagt aagtttggtt gataaattca    360
aagagaagct ccctgcacaa cctaacaagg gggaggggga ggagaaagtg gaagtggaga    420
```

```
agaaagcagg tgtcaaagaa aagctccctg tacaacctaa caagggggaa agggaagaga     480
aagcggaagt ggagaagaaa gtaggtttgc tagataaaat taaagagaag ctacccggac     540
actctaacca aaaaggaagg ggaagggaaa agaagaagaa gaagcagaag tggcgaaaaa     600
ggcgagtcct gatcgataaa atcaaagaga aactccccgg acatcatacc aagaaggaag     660
gagaagagga agaaaagaag caaaactatt aaagctactg taaacaagat cttgtccaag     720
tctatattta actcgttcct tactttgcct gagagcattg ataggaatga tgatatgtat     780
ctgttgtgtg gttttgatta atgatttgcg ttagaagaga agataaaagt tatgaatgtg     840
agccgacgga tccacattgg catctgcagt attgcatatt tcatgaacgg ggttccaatt     900
accttacccg ggtcctgagt gttcggtcaa gttattatca tatatttatg gacattttta     960
tatgtggcaa caaggattgc ttgggatcca ggaagtaagc aggatttcat taatcatgaa    1020
ttgtatctgg ccttttgagt tgcaatatct tttggctgga attggcaact tcttcctgag    1080
tgtggtgttg tagttatgta tgagaggatc atatggatat gtaaagtccg tcataaaact    1140
taa                                                                   1143
```

<210> 11
<211> 222
<212> PRT
<213> Pinus sylvestris

<400> 11

```
Met Arg Arg Leu Arg Met Arg Leu Ala Ser Tyr Tyr Pro Ala Cys Ser
1               5                   10                  15

Ser Ala Trp Ser Arg Thr Arg Pro Met Val Thr Arg Gly Asn Phe Thr
            20                  25                  30

Pro Glu Glu Ala Glu Glu Lys Lys His Thr Gly Leu Ile Gly Lys Leu
        35                  40                  45

His Arg Thr His Ser Ser Ser Ser Ser Ser Ser Ser Asp Glu Glu Glu
    50                  55                  60

Glu Gly Glu Lys Lys Lys Glu Lys Glu Arg Lys Lys Lys Glu Ala Lys
65                  70                  75                  80

Asp Lys Thr Asn Lys Lys Leu Pro Gly Asp Gly Gln His Ser Ser Asp
                85                  90                  95
```

Gln Ser Gly Ile Glu Glu Glu Lys Lys Val Ser Leu Val Asp Lys Phe
100 105 110

Lys Glu Lys Leu Pro Ala Gln Pro Asn Lys Gly Glu Gly Glu Glu Lys
115 120 125

Val Glu Val Glu Lys Lys Ala Gly Val Lys Glu Lys Leu Pro Val Gln
130 135 140

Pro Asn Lys Gly Glu Arg Glu Glu Lys Ala Glu Val Glu Lys Lys Val
145 150 155 160

Gly Leu Leu Asp Lys Ile Lys Glu Lys Leu Pro Gly His Ser Asn Gln
165 170 175

Lys Gly Arg Gly Arg Glu Lys Lys Lys Lys Lys Gln Lys Trp Arg Lys
180 185 190

Arg Arg Val Leu Ile Asp Lys Ile Lys Glu Lys Leu Pro Gly His His
195 200 205

Thr Lys Lys Glu Gly Glu Glu Glu Glu Lys Lys Gln Asn Tyr
210 215 220

<210> 12
<211> 108
<212> PRT
<213> Pinus sylvestris

<400> 12

Gly His Gly His Glu Gly Gln Phe Ala Arg Glu Glu Ala Glu Gln Gln
1 5 10 15

Lys His Thr Gly Lys Leu His Arg Ser Asn Ser Ser Ser Ser Ser Ser
20 25 30

Ser Ser Asp Glu Glu Glu Glu Gly Arg Lys Lys Lys Glu Gly Gly Arg
35 40 45

Lys Lys Lys Gly Ser Lys Asp Lys Thr Lys Glu Lys Leu Pro Gly Asp
50 55 60

```
Gly Lys Gln Tyr Pro Gly Glu His Glu Lys Gln Tyr Ser Arg Gln His
65                  70                  75                  80

Gly Gly Glu Glu Glu Lys Lys Ala Gly Met Leu Asp Lys Ile Lys Ala
                85                  90                  95

Lys Leu Pro Gly Gln Asn Lys Asn Asp Gly Glu Gln
            100                 105
```

<210> 13
<211> 93
<212> PRT
<213> Pinus sylvestris

<400> 13

```
Met Ala Gln Pro Ala His Gln Asp Arg Gly Met Phe Gly Lys Lys Lys
1               5                   10                  15

Glu Asp Glu Gly Lys Gln Cys Asp Gln Thr Glu Gly Gly Arg Cys Glu
            20                  25                  30

Glu Gly Lys Lys Pro Gly Met Val Asp Lys Ile Lys Glu Lys Ile Pro
        35                  40                  45

Gly Gln Gln Gln Lys Leu Pro Gly Asp Gly Lys Gln Cys Cys Gly Gln
    50                  55                  60

Pro Gly Gly Glu Glu Glu Lys Lys Pro Gly Met Val Asp Lys Ile Lys
65                  70                  75                  80

Gln Lys Leu Pro Gly Gln Gln Asn Lys Thr Gly Cys Asp
                85                  90
```

<210> 14
<211> 93
<212> PRT
<213> Pinus sylvestris

<400> 14

```
Met Ala Gln Pro Ala His Gln Asp Arg Gly Met Phe Gly Lys Lys Lys
1               5                   10                  15

Glu Asp Glu Gly Arg Gln Cys Asp Gln Thr Glu Gly Gly Arg Cys Glu
            20                  25                  30
```

```
Glu Gly Lys Lys Pro Gly Met Val Asp Lys Ile Lys Glu Lys Ile Pro
        35                  40                  45

Gly Gln Gln Gln Lys Leu Pro Gly Asp Gly Lys Gln Cys Gly Gly Gln
    50                  55                  60

Pro Gly Arg Glu Glu Glu Lys Lys Pro Gly Met Val Asp Lys Ile Lys
65                  70                  75                  80

Gln Lys Leu Pro Gly Gln Gln Asn Lys Asn Asp Val Glu
                85                  90
```

<210> 15
<211> 95
<212> PRT
<213> Pinus sylvestris

<400> 15

```
Met Ala Glu Glu Gln Gln Asp Arg Gly Met Phe Gly Leu Leu Gly Lys
1               5                   10                  15

Lys Lys Glu Asp Glu Gly Lys Gln Cys Asp Gln Thr Glu Gly Gly Arg
            20                  25                  30

Cys Glu Glu Gly Lys Lys Pro Gly Met Val Asp Lys Ile Lys Glu Lys
        35                  40                  45

Ile Pro Gly Gln Gln Gln Lys Leu Pro Gly Asp Gly Lys Gln Cys Gly
    50                  55                  60

Gly Gln Pro Gly Arg Glu Glu Glu Lys Lys Pro Gly Met Val Asp Lys
65                  70                  75                  80

Ile Lys Gln Lys Leu Pro Gly Gln Gln Asn Lys Asn Asp Val Glu
                85                  90                  95
```

<210> 16
<211> 95
<212> PRT
<213> Pinus sylvestris

<400> 16

```
Met Ala Glu Glu Gln His Asp Arg Gly Met Phe Gly Leu Leu Cys Lys
1               5                   10                  15
```

```
Lys Lys Glu Asp Glu Gly Lys Gln Cys Asp His Thr Glu Gly Gly Arg
            20                  25                  30

Cys Glu Glu Gly Lys Lys Pro Gly Met Val Asp Lys Ile Lys Glu Lys
        35                  40                  45

Ile Leu Gly Gln Gln Pro Lys Leu Pro Arg Asp Arg Lys Gln Cys Gly
    50                  55                  60

Cys His Pro Gly Arg Val Glu Glu Lys Arg Pro Arg Met Val Asp Lys
65                  70                  75                  80

Ile Lys Gln Lys Leu Pro Gly His Gln Asn Lys Asn Asp Val Glu
                85                  90                  95
```

<210> 17
<211> 95
<212> PRT
<213> Pinus sylvestris

<400> 17

```
Met Ala Glu Glu Gln Gln Asp Arg Gly Met Phe Gly Leu Leu Gly Lys
1               5                   10                  15

Lys Lys Glu Asp Glu Gly Lys Gln Cys Asp Gln Thr Glu Gly Gly Arg
            20                  25                  30

Cys Glu Glu Gly Lys Lys Pro Gly Met Val Asp Lys Val Lys Glu Lys
        35                  40                  45

Ile Pro Gly Gln Gln Gln Lys Leu Pro Gly Asp Gly Lys Gln Cys Cys
    50                  55                  60

Gly Gln Pro Gly Gly Glu Glu Glu Lys Lys Pro Gly Met Val Asp Lys
65                  70                  75                  80

Ile Lys Gln Lys Leu Pro Gly Gln Gln Asn Lys Thr Gly Cys Asp
                85                  90                  95
```

<210> 18
<211> 94
<212> PRT
<213> Pinus sylvestris

<400> 18

```
Met Ala Glu Glu Gln Gln Asp Arg Gly Met Phe Gly Leu Leu Gly Lys
1               5                   10                  15

Lys Lys Glu Asp Glu Gly Lys Gln Cys Asp Gln Gly Pro Pro Arg Thr
            20                  25                  30

Glu Val Gly Arg Cys Glu Glu Gly Lys Lys Pro Gly Met Val Asp Lys
        35                  40                  45

Ile Lys Glu Lys Ile Pro Gly Gln Gln Gln Lys Leu Pro Gly Asp Gly
    50                  55                  60

Lys Gln Cys Gly Gly Gln Pro Gly Arg Glu Glu Glu Lys Lys Pro Gly
65                  70                  75                  80

Met Val Asp Lys Ile Lys Gln Lys Leu Pro Gly Gln Gln Asn
                85                  90
```